(19) Europäisches Patentamt
European Patent Office
Office européen des brevets

(11) EP 1 818 400 A1

(12) EUROPEAN PATENT APPLICATION
published in accordance with Art. 158(3) EPC

(43) Date of publication:
**15.08.2007 Bulletin 2007/33**

(21) Application number: **05811383.8**

(22) Date of filing: **22.11.2005**

(51) Int Cl.:
***C12N 15/09*** *(2006.01)* ***C07K 16/40*** *(2006.01)*
***C12N 9/00*** *(2006.01)* ***C12P 19/34*** *(2006.01)*
***C12P 21/02*** *(2006.01)* ***C12Q 1/25*** *(2006.01)*

(86) International application number:
**PCT/JP2005/021938**

(87) International publication number:
**WO 2006/054807 (26.05.2006 Gazette 2006/21)**

(84) Designated Contracting States:
**CH DE FR GB LI**

(30) Priority: **22.11.2004 JP 2004338178**

(71) Applicant: **Gifu University**
**Gifu-shi**
**Gifu 5011193 (JP)**

(72) Inventors:
- **NISHIKAWA,Kazuya**
**5011193 (JP)**
- **YOKOGAWA, Takashi**
**5011193 (JP)**
- **OHNO, Satoshi**
**5011193 (JP)**

(74) Representative: **Rapp, Bertram et al**
**Charrier Rapp & Liebau**
**Patentanwälte**
**Postfach 31 02 60**
**86063 Augsburg (DE)**

# (54) METHOD OF SITE-SPECIFICALLY INTRODUCING NON-NATURAL AMINO ACID INTO PROTEIN USING MITOCHONDRIAL PROTEIN AND METHOD FOR EFFECTIVELY PREPARING tRNA

(57) This invention is intended to provide a protein synthesis system used for producing a tryptophan analogue-containing non-natural-amino-acid-containing protein that satisfies the following conditions: (i) tRNA that transfers a non-natural amino acid is not recognized by an endogenous aminoacyl tRNA synthetase (aaRS); (ii) it is recognized selectively by aaRS exclusive for a non-natural amino acid; and (iii) endogenous tRNA is not recognized by aaRS exclusive for a non-natural amino acid. In the eukaryotic organism-derived cell-free protein synthesis system, a yeast mitochondrial tryptophanyl tRNA synthetase is used in combination with mitochondrial $tRNA^{Trp}$ (mt $tRNA^{Trp}$).

EP 1 818 400 A1

## Description

Technical Field

The present invention relates to a mitochondrial aminoacyl tRNA synthetase. More particularly, the present invention relates to a yeast mitochondrial tryptophanyl tRNA synthetase that is capable of aminoacylating yeast mitochondrial $tRNA^{Trp}$.

Further, the present invention relates to a method for producing a protein containing a non-natural amino acid with the utilization of a yeast mitochondrial aminoacyl tRNA synthetase.

Also, the present invention relates to preparation of RNA. More particularly, the present invention relates to a method for preparing tRNA capable of aminoacylating tRNA.

Background Art

1. Aminoacyl tRNA synthetase

An aminoacyl tRNA synthetase exists in every organism. In protein synthesis, it utilizes the energy of ATP hydrolysis to activate and allow amino acids to bind to transfer RNA (tRNA). In prokaryotic organisms, 20 aminoacyl tRNA synthetase species are present, relative to 20 natural amino acid species. In eukaryotic organisms, 20 aminoacyl tRNA synthetase species are present in the cytoplasm and 20 different aminoacyl tRNA synthetase species are present in the mitochondria. Such aminoacyl tRNA synthetase species are roughly classified into class I and class II. Class I comprises in its amino acid sequence a signature sequence referred to as HXGH and a nucleotide binding region of KMSK (Rossmann-fold domain). Class I includes aminoacyl tRNA synthetases that react with arginine, cysteine, glutamine, glutamic acid, isoleucine, leucine, methionine, tryptophan, tyrosine, and valine.

Class II does not include such characteristic sequences but includes aminoacyl tRNA synthetase that independently activates alanine, asparagin, aspartic acid, glycine, lysine, phenylalanine, proline, and serine.

An aminoacyl tRNA synthetase that activates each such amino acid catalyzes a process of allowing an amino acid to bind to tRNA. In the case of a tyrosyl-tRNA synthetase, for example, the reaction proceeds as follows: Tyr+ATP+$tRNA^{Tyr}$+ tyrosyl-tRNA synthetase + $\rightarrow$ AMP+Tyr-$tRNA^{Tyr}$+ tyrosyl-tRNA synthetase.

Up to the present, aminoacyl RNA synthetases have been sampled from various organisms for each amino acid, and such synthetases have been reported.

Examples of known tryptophanyl tRNA synthetases include those derived from *E. coli* (JBC Vol. 257, no. 11, pp. 6132-6136) and those derived from *Bacillus subtilis* (JBC Vol. 264, pp. 4304-4311).

Also, aminoacyl-tRNA synthetases derived from microorganisms are used as targets for new antibiotics. Further, specific antibodies reacting therewith can also be utilized.

2. Method for producing protein containing non-natural amino acid

A method was developed that uses suppressor tRNA and uses one (amber codon) of the 3 natural termination codons as a non-natural amino acid codon to site-specifically incorporate a non-natural amino acid (Science, 244, 182-188, 1989). In this method, a codon at a site in the gene into which introduction of a non-natural amino acid is intended is mutated into a codon that corresponds to suppressor tRNA. Such mutant gene is used to perform cell-free protein translation in the presence of tRNA comprising a non-natural amino acid bound thereto, which was prepared via a chemical bond between suppressor tRNA and a non-natural amino acid. Thus, a protein containing a non-natural amino acid was prepared.

Thereafter, tRNA comprising a non-natural amino acid chemically bound to suppressor tRNA was injected into Xenopus oocytes using the mutant gene, in order to synthesize a protein that contains a non-natural amino acid in a site-specific manner (JBC vol. 271, pp. 23169).

The method involving the use of suppressor tRNA suffers from the limited amount of non-natural amino acids introduced. As a method that is intended to overcome such a drawback, a method wherein non-natural amino acids are bound to parts of various tRNAs is available (JP Patent No. 3317983). In this production method, some of isoaccepting tRNAs corresponding to given types of amino acids are selectively bound to non-natural amino acids and the resultants are subjected to protein synthesis using protein-constituting amino acids in the cell-free protein synthesis system, in order to incorporate non-natural amino acids into given codon groups.

In recent years, a given type of aminoacyl tRNA synthetase is used in combination with tRNA to synthesize non-natural-amino-acid-containing proteins (hereafter they may be referred to as "non-natural amino acid proteins") while reutilizing tRNA. Further, non-natural-amino-acid-containing proteins are synthesized in *E. coli* using a mutant enzyme, which was prepared from tyrosyl-tRNA synthetase derived from *Methanococcus* that had been mutated specifically for non-natural amino acid, and tRNA corresponding thereto (Science vol. 292, pp. 498-500).

The present inventors have also invented a method for protein synthesis wherein a template is added to a protein synthesis system comprising a tyrosine analogue, suppressor tRNA, and a mutant of aminoacyl tRNA synthetase that binds the tyrosine analogue to the suppressor tRNA. In this method, azidotyrosine, acetyltyrosine, aminotyrosine, or DOPA can be introduced as a tyrosine analogue (JP Patent Publication (kokai) No. 2004-261160 (A)).

Up to the present, protein X-ray crystal structure analysis has involved the use of a selenomethionine-substituted protein produced by the method disclosed in Toshiyuki Shimizu, Kengo Okada, and Toshio Hakoshima, Chapter 17, X-ray crystal structural analysis, Expression and preparation of selenomethionine-substituted protein, "KISO SEIKAGAKU JIKKENHO (Basic Biochemical Experiment) 3, Tanpakushitsu (Protein) I. Kenshutu (detection)·Kouzou kaiseki hou (structural analysis)," Tokyo Kagaku Dojin, version 1, edition 1, February 15, 2001, pp. 189-190. When producing such selenomethionine-substituted protein, at the outset, a plasmid comprising a methionine auxotroph of E. *coli* and the gene of the target protein inserted therein is prepared for the purpose of transformation, and an antibiotic-containing agarose gel plate is used to isolate a single colony that expresses the target protein. Subsequently, the isolated transformant is cultured in a medium containing selenomethionine instead of methionine to express and purify the target protein.

3. tRNA

The correlation between DNA as genetic information and a functional protein was demonstrated as the idea of the "central dogma." In this idea, a process of producing a protein from DNA is divided into 2 steps. In the first step, "a given region (gene) of DNA is copied into a form (RNA (mRNA)) that can be used for protein synthesis." In the second step, "RNA is used to produce a protein." In the first step, the DNA double helix is unwound, and one of the DNA strands is used as a template to prepare an RNA strand (RNA prepared herein is particularly referred to as messenger RNA (mRNA)). This process is referred to as "transcription." In the second step, nucleic acid information, i.e., mRNA, is substituted with an amino acid to synthesize a protein, and this process is referred to as "translation." Three nucleotides (triplet) on mRNA containing genetic information correspond to a single amino acid. This triplet is referred to as a "codon," it gives 4 x 4 x 4 = 64 possible combinations, and it specifies 20 different amino acid species and a "termination codon" indicating the termination of protein synthesis. This can be summarized in the list of genetic codes. Several codons specify a single amino acid; however, methionine uses only one codon, for example. Specifically, such methionine codon (AUG) is an "initiation codon" indicating the initiation of protein synthesis. A molecule that bridges the codon and the amino acid is transfer RNA (tRNA), which is indispensable for translation of genetic codes.

Disclosure of the Invention

A mitochondrial aminoacyl tRNA synthetase is an important target of development with regard to pharmaceutical products such as antibiotics.

As mentioned above, a method involving the use of suppressor tRNA and the aminoacyl tRNA synthetase that binds the amino acid analogue to the suppressor tRNA in the protein synthesis system is preferable in terms of effective production of non-natural amino acid proteins. Up to the present, however, types of non-natural amino acids that can be introduced are limited to, for example, tyrosine analogues or alanine analogues.

Accordingly, (1) the first object of the present invention is to provide a novel aminoacyl tRNA synthetase.

Further, (2) the second object of the present invention is to provide a novel non-natural-amino-acid-containing protein synthesis system, wherein suppressor tRNA and an aminoacyl tRNA synthetase that binds the amino acid analogue to the suppressor tRNA are used to introduce amino acid mutants other than tyrosine into a protein.

Among amino acids, the ability of effectively introducing a mutant is important for tryptophan, which can be fluorescently labeled. Accordingly, the present invention is further intended to provide a tryptophanyl tRNA synthetase and a protein synthesis system involving the use of suppressor tRNA and a tryptophanyl tRNA synthetase that binds a tryptophan analogue to the suppressor tRNA.

The method involving the use of suppressor tRNA and an aminoacyl tRNA synthetase that binds the amino acid analogue to the suppressor tRNA for the protein synthesis system is required to satisfy the following conditions. That is, (i) tRNA that transfers a non-natural amino acid is not recognized by an endogenous aminoacyl tRNA synthetase (aaRS), (ii) it should be recognized selectively by aaRS exclusively used for a non-natural amino acid, and (iii) endogenous tRNA should not be recognized by aaRS exclusively used for a non-natural amino acid. Thus, the present invention is further intended to provide a system that satisfies the above conditions in the production of non-natural-amino-acid-containing proteins containing tryptophan analogues.

At present, tRNA preparation employs *in vitro* transcription involving the use of template DNA and a RNA polymerase. In this method, however, guanosine (G) is preferably used to initiate transcription. Accordingly, it was difficult to prepare tRNA comprising, as the first nucleotide, a nucleotide other than G. In order to overcome such drawback, a preparation method involving the use of an artificial enzyme comprising RNA (ribozyme) was reported within the last

few years, although such method was not satisfactorily efficient.

The present inventors isolated a gene encoding a yeast mitochondrial tryptophanyl tRNA synthetase and actually expressed such gene, in order to isolate a novel and active tryptophanyl tRNA synthetase.

Further, the present inventors used the enzyme of the present invention in combination with tryptophan $tRNA^{Trp}$ in the cell-free protein synthesis system derived from a eukaryotic organism. Accordingly, the present inventors discovered that a non-natural-amino-acid-containing protein that would satisfy the following conditions and that could contain a tryptophan analogue in a site-specific manner could be prepared. That is, (i) tRNA that transfers a non-natural amino acid is not recognized by an endogenous aminoacyl tRNA synthetase (abbreviated as "aaRS"), (ii) it is recognized selectively by aaRS exclusively used for a non-natural amino acid, and (iii) endogenous tRNA is not recognized by aaRS exclusively used for a non-natural amino acid.

Furthermore, the present inventors constructed a mass-expression system for RNaseP derived from E. *coli* and discovered that tRNA having a sequence comprising as the first nucleotide a nucleotide other than G could be prepared.

This description includes part or all of the contents as disclosed in the description and/or drawings of Japanese Patent Application No. 2004-338178, which is a priority document of the present application.

Brief Description of the Drawings

Fig. 1 shows the results of confirmation of mtWRS gene amplification via 1% agarose gel electrophoresis.
Fig. 2 shows the results of confirmation of incorporation of the mtWRS gene, following the ligation with pUC19.
Fig. 3 shows the results of confirmation of subcloning of the mtWRS gene into the pYC2/CT vector.
Fig. 4 shows the results of confirmation of mtWRS expression using pYC2WRS, insertion of the mtWRS gene into which had been confirmed.
Fig. 5 shows the results of confirmation of C5 protein expression using pETC5, insertion of the C5 protein gene into which had been confirmed.
Fig. 6 shows the results of confirmation of preparation of M1 RNA.
Fig. 7 shows the results of confirmation of activation by the C5 protein and M1 RNA.
Fig. 8 shows the results of confirmation of mitochondrial $tRNA^{Trp}$ preparation.
Fig. 9 shows the results of confirmation of preparation of cytoplasmic $tRNA^{Trp}$ and a mutant thereof.
Fig. 10 shows the results of confirmation of $TRNA^{Tyr}$ preparation.
Fig. 11 shows the results of confirmation of the capacity of tRNA for accepting $^{14}C$- tryptophan using mtWRS and mt $tRNA^{Trp}$.
Fig. 12 shows electrophoresis for identifying a signal peptide cleavage site of mtWRS.
Fig. 13 shows the results of confirmation of the cross-reaction between mtWRS and mt $tRNA^{Trp}$ using a wheat germ extract (S100- fraction, without nucleic acid) and wheat tRNAmix.
Fig. 14 shows the results of confirmation of suppression of an opal codon in the cell-free protein synthesis system derived from the wheat germ extract.
Fig. 15 shows the amino acid pocket of a crystal structure of B. *stearothermophilus* TrpRS, and the information in parentheses represents mtWRS residues.
Fig. 16 shows the results of confirmation of acceptance of tryptophan and a tryptophan analogue into mt $tRNA^{Trp}$ with the aid of mtWRS and F38A by the formation of a triple complex using EF-Tu of *T. thermophilus.*
Fig. 17 shows the results of confirmation of acceptance of tryptophan and a tryptophan analogue into mt $tRNA^{Trp}$.

Best Modes for Carrying out the Invention

I. Mitochondrial tryptophanyl tRNA synthetase

1. Novel mitochondrial tryptophanyl tRNA synthetase

The present inventors deduced the amino acid sequence of the mitochondrial tryptophanyl tRNA synthetase. As a result, they obtained the amino acid sequence as shown in SEQ ID NO: 1. Based on the prediction using a common signal peptide prediction program that the sequence would be cleaved at position 19, 22, or 24, they intended to express the mitochondrial tryptophanyl tRNA synthetase in E. *coli* via genetic engineering. However, they only obtained insoluble proteins, solubilization efficiency was very poor, and they could not recover active proteins. As a result of research, the present inventors discovered that a signal peptide was comprised of nucleotides 1 to 15 of the sequence as shown in SEQ ID NO: 1, and they succeeded in designing and obtaining active soluble proteins based thereon.

The present invention includes a mitochondrial tryptophanyl tRNA synthetase derived from *Saccharomyces cerevisiae* comprising amino acid 16 and its downstream region of the amino acid sequence as shown in SEQ ID NO: 1, i.e., a mitochondrial tryptophanyl tRNA synthetase derived from *Saccharomyces cerevisiae* comprising the sequence as shown in SEQ ID NO: 2.

Further, the present invention includes the following polypeptides.

(1) A polypeptide comprising part, and at least a region comprising the N terminus to the residue 237, of the amino acid sequence as shown in SEQ ID NO: 2, which has the activity of the mitochondrial tryptophanyl tRNA synthetase.

(2) A polypeptide comprising an amino acid sequence derived from the amino acid sequence as shown in SEQ ID NO: 2 by substitution, addition, and/or deletion of 1 or several (for example, 1 to 50, preferably 1 to 30, and more preferably 1 to 10) amino acids and ISTVQ as a region comprising 5 amino acid residues from the N-terminus, which has the activity of the mitochondrial tryptophanyl tRNA synthetase.

(3) A polypeptide comprising ISTVQ as a region comprising 5 amino acid residues from the N-terminus and having 75% or higher, preferably 85% or higher, and more preferably 95% or higher identity with the sequence as shown in SEQ ID NO: 2, which has the activity of the mitochondrial tryptophanyl tRNA synthetase.

2. Mitochondrial tryptophanyl tRNA synthetase gene

Examples of genes encoding the mitochondrial tryptophanyl tRNA synthetase of the present invention include a nucleic acid comprising the nucleotide sequence as shown in SEQ ID NO: 3 and a nucleic acid comprising a sequence complementary thereto. A particularly preferable example of such gene is a nucleic acid comprising a nucleotide sequence comprising nucleotide 45 and the downstream region of the nucleic acid sequence as shown in SEQ ID NO: 3 or a complementary sequence thereof.

A nucleic acid that hybridizes under stringent conditions to the nucleic acid as shown in SEQ ID NO: 3 or a nucleic acid complementary thereto and that encodes a polypeptide having the activity of the tryptophanyl tRNA synthetase is also within the scope of the present invention. As stringent conditions, common stringent conditions may be employed. For example, Hybond N+ nylon membrane (Amersham Pharmacia) that had been treated at 120°C for 20 minutes to bind DNA thereto is treated in a phosphate buffer (0.5M $Na_2HPO_4$, 1 mM EDTA, and 7% SDS) at 65°C for 5 minutes for prehybridization. Hybridization is carried out with the addition of the gene comprising the sequence as shown in SEQ ID NO: 3 to the above buffer at 65°C for 17 hours. After hybridization, the membrane is treated at room temperature for 20 minutes in 2x SSC containing 0.1% SDS (standard saline citrate; 1x SSC comprises 150 mM NaCl and 15 mM sodium citrate; pH 7.0), followed by washing twice in 1x SSC containing 0.1% SDS at 65°C for 20 minutes. Further, the membrane is treated in 0.1x SSC containing 0.1% SDS at 65°C for 20 minutes to complete the washing. X-ray film exposure is carried out at -80°C for 24 hours.

3. Preparation of mitochondrial tryptophanyl tRNA synthetase

The mitochondrial tryptophanyl tRNA synthetase can be prepared in the following manner.

The gene comprising the sequence as shown in SEQ ID NO: 3 or 4 is incorporated into a known expression vector, and the expression vector is introduced into an adequate host cell to express the mitochondrial tryptophanyl tRNA synthetase gene. Thus, the mitochondrial tryptophanyl tRNA synthetase can be prepared.

As host cells, any host cells for gene expression can be used. For example, bacteria such as E. *coli,* insect cells, animal cells such as COS or CHO, and, preferably, yeast cells can be used.

An expression vector is not particularly limited. For example, a vector that can express a protein provided with a purification means such as an His-tag is preferably used. When an E. *coli* host cell is used, the pET host such as pET101, pGEX (Amersham Pharmacia), or pRSET (Invitorgen) can be used. When animal cells such as COS or CHO are used as host cells, pXM, pDC201, pCI, or the like can be used. When insect cells are used as host cells, a baculovirus expression vector can be used. When yeast host cells are used, a yeast expression vector such as pPICZα and a pYC vector such as pYC2/Ct can be used. Such vector is transduced into a host cell via a known means. In the case of an inducible expression vector, expression of the target mitochondrial tryptophanyl tRNA synthetase gene is induced to produce a mitochondrial tryptophanyl tRNA synthetase. When the produced mitochondrial tryptophanyl tRNA synthetase is designed to be secreted extracellulary, the mitochondrial tryptophanyl tRNA synthetase is recovered from the extracellular culture solution. When the produced mitochondrial tryptophanyl tRNA synthetase is accumulated intracellularly, the host cell is disrupted to recover the mitochondrial tryptophanyl tRNA synthetase. The recovered mitochondrial tryptophanyl tRNA synthetase is then purified via a combination of known techniques. For example, an antibody reacting with the mitochondrial tryptophanyl tRNA synthetase is prepared and the mitochondrial tryptophanyl tRNA synthetase is then purified with the use of such antibody. When the protein is His-tagged, for example, Ni-NTA can be used.

The mitochondrial tryptophanyl tRNA synthetase gene comprising the sequence as shown in SEQ ID NO: 4 is

incorporated into a high-performance expression vector for *E. coli,* and the gene is then introduced into E. *coli,* which enables mass-production. A specific example is a pET vector (pET21-a(+) (Novagen)).

4. Mitochondrial tryptophanyl tRNA synthetase mutant, gene thereof, and method for producing the same

The mitochondrial tryptophanyl tRNA synthetase of the present invention can be subjected to point mutation so that the tryptophan analogue can further be bound to tRNA.
For example, phenylalanine 38 and valine 193 of SEQ ID NO: 1 are designated as targets of modification and substituted with alanine or glycine. Thus, recognition of a substrate amino acid (tryptophan) can be deteriorated and recognition of the tryptophan analogue can be enhanced.

5. Inhibitor, antibody, and pharmaceutical product against mitochondrial tryptophanyl tRNA synthetase

An antibody against the mitochondrial tryptophanyl tRNA synthetase can be prepared by a known technique.
For example, a monoclonal antibody against the mitochondrial tryptophanyl tRNA synthetase can be prepared in the following manner. Preferably, the purified mitochondrial tryptophanyl tRNA synthetase can be mixed with an adequate adjuvant, such as the Freund's complete or incomplete adjuvant, and then used as an immunogen.
Examples of animals to be immunized include mammalian animals such as rats and mice. Preferably, mice (BALB/c) or rats can be used.
The target animals for immunization are inoculated with immunogens. Methods of immunization can be determined in accordance with the animal species, the amount of antigens, the necessity of an adjuvant, the number of times of immunization, and other conditions. In the case of mice, for example, an antigen may be mixed with an adjuvant and the resultant can be injected subcutaneously, intravenously, or intraperitoneally. According to need, a booster can be provided 1 to 4 weeks after the initial immunization.
When the final immunization is carried out 4 to 10 weeks after the initial immunization, B cells are sampled from, for example, the spleens of the immunized animals 3 or 4 days after the final immunization.
The sampled immunized B cells are fused with myeloma cells by a known method such as the Milstein's technique.
Examples of mouse myeloma cells include P3-X63Ag8(X63), P3/NS1/1-Ag4-1, P3X63Ag8U1, P3X63Ag8.653, Sp2/O-Ag14, and Sp/O/Fo-2. Examples of rat myeloma cells include Y-Ag1.2.3, YB/O, and IRF983F.
Immunized spleen cells (B cells) are fused to myeloma cells at lto 10, for example. Cell fusion can be carried out using a known agent for cell fusion, such as polyethylene glycol (30% to 40%) or HVJ (hemagglutinating virus of Japan). Fused cells can be selected using, for example, HAT medium.
From among the selected fused cells, fused cells that produce the target monoclonal antibodies can be further selected.
The mitochondrial tryptophanyl tRNA synthetase of the present invention is very useful as a antibiotic target, and it can be used for screening for novel antibiotics or a novel pharmaceutical product.
Specifically, activity of the mitochondrial tryptophanyl tRNA synthetase is assayed in the presence of a candidate compound, and a candidate compound that lowers the activity of the mitochondrial tryptophanyl tRNA synthetase can be selected. Activity of the mitochondrial tryptophanyl tRNA synthetase can be assayed using the mitochondrial tryptophanyl tRNA synthetase (mtWRS) and mt $TRNA^{Trp}$ to detect the capacity of tRNA for accepting $^{14}C$-tryptophan, for example.

II. Non-natural-amino-acid-containing protein synthesis system

1. Non-natural-amino-acid-containing protein synthesis system involving the use of mitochondrial tryptophanyl tRNA synthetase in combination with tryptophan $tRNA^{Trp}$

(1) A method wherein a protein-encoding gene or a mutant gene or mutant mRNA in which a codon encoding an amino acid at a given site of mRNA is mutated into a termination codon and aminoacyl tRNA synthetase and amino acid analogue suppressor tRNA are used to produce a non-natural amino acid protein comprising such amino acid analogue; and (2) a method wherein mRNA transcribed from a protein-encoding gene, an aminoacyl tRNA synthetase, and RNA are used to allow the presence of excess amino acid analogues to produce a non-natural amino acid protein containing such amino acid analogues, and other methods are available.
In the case of the method (1) above, (i) when a natural amino acid is bound to suppressor tRNA with the aid of the aminoacyl tRNA synthetase endogenous to the protein synthesis system to be used, the non-natural amino acid protein of interest cannot be obtained, and (ii) the aminoacyl tRNA synthetase for binding the added non-natural amino acid to tRNA must be designed so as not to bind a non-natural amino acid to endogenous tRNA. In other words, the

aminoacyl tRNA synthetase and amino acid analogue suppressor tRNA to be added should not undergo a cross reaction with the endogenous aminoacyl tRNA synthetase and tRNA of the protein synthesis system. If a system that would not cause such cross reaction could be developed, such system could be satisfactorily used for the synthesis of non-natural amino acid proteins.

The present inventors tested a combination of the mitochondrial aminoacyl tRNA synthetase and tRNA, and particularly a combination of the tryptophanyl tRNA synthetase and tryptophan $tRNA^{Trp}$ or suppressor tRNA, as a combination of the aminoacyl tRNA synthetase or tRNA that would not undergo a cross reaction with aminoacyl tRNA synthetases and tRNA of the endogenous protein synthesis system. As a result, they discovered that it would not cause a cross reaction with the cytoplasmic tryptophanyl tRNA synthetase and tryptophan $tRNA^{Trp}$. In particular, mitochondrial $tRNA^{Trp}$ sometimes recognizes UGA because of the fluctuation, in addition to UGG. Also, the tryptophanyl tRNA synthetase is considered to effectively bind tryptophan or a tryptophan analogue to suppressor tRNA where a suppressor tRNAthat recognizes an opal codon is used as suppressor tRNA.

Specifically, the present invention includes a method for producing a non-natural amino acid-containing polypeptide by mutating an amino acid at a given site of a protein into a non-natural amino acid, wherein a mutant gene in which the amino acid codon at a given site of the protein-encoding gene has been mutated into an opal codon, mitochondrial tryptophanyl tRNA synthetase (mtWRS), and mitochondrial $tRNA^{Trp}$ or mitochondrial suppressor $tRNA^{Opal}$ is added to a protein translation/synthesis system to produce the non-natural amino acid-containing polypeptide.

2. Fundamental protein synthesis system

The present invention includes a method for synthesizing natural amino acid proteins involving the use of the mitochondrial tryptophanyl tRNA synthetase (hereafter abbreviated as "mtWRS") and mitochondrial $tRNA^{Trp}$ (hereafter abbreviated as "mt $tRNA^{Trp}$").

A cell-free protein expression system or a protein synthesis system involving the use of plant cells, animal cells, insect cells, and other eukaryotic cells can be used as a protein expression system used for the method for synthesizing non-natural amino acid proteins containing such mtWRS and mt $tRNA^{Trp}$. In the method for synthesizing non-natural amino acid proteins of the present invention, use of the protein synthesis system from E. *coli* is not suitable. However, such method can use a cell extract, which is obtained by modifying the E. *coli* genome and gene and eliminating a factor that negatively affects non-natural amino acid introduction (e.g., tryptophanyl tRNA synthetase or $tRNA^{Trp}$) therefrom via disruption (deletion), modification of substrate specificity, or immunoprecipitation.

As a cell-free protein synthesis system, an *in vitro* translation system that performs translation from template mRNA or *an in vitro* transcription-translation system that performs transcription and translation from template DNA can be employed. As such *an in vitro* protein synthesis system, a system that is prepared by disrupting cells, adding 20 amino acid species (mainly S30) and ATP and GTP as energy sources, and further adding an energy-regenerating system, has been used in the past. In particular, a system that is prepared from E. *coli,* wheat germ, or rabbit reticulocyte has been extensively used. In the present invention, a system prepared from rabbit reticulocyte, and particularly preferably a system prepared from a wheat germ extract, can be used.

In recent years, a system that is constructed by purifying components that are necessary for translation and for transcription according to need and mixing them can also be used. Alternatively, a system prepared by combining the aforementioned systems can also be used.

Regarding a system involving the use of cultured cells or the like, Chinese hamster ovarian (CHO) cells, a baculovirus vector protein expression system, Xenopus oocytes, and the like are preferably used. Also, use of a cell extract from which factors that negatively affect non-natural amino acid introduction (e.g., introduction of a nuclease, a protease, a release factor, or an oxidoreductase) have been eliminated via disruption (deletion), immunoprecipitation, or other means is also preferable.

3. Combination of mtWRS and mt tRNA

mtWRS and mt $tRNA^{Trp}$ that can be used for the non-natural amino acid protein synthesis system of the present invention are preferably derived from the same species.

(A) Various types of mtWRS can be used, provided that the mtWRS is mitochondrial tryptophanyl tRNA synthetase. Preferable examples thereof include the following.

(1) A mitochondrial tryptophanyl tRNA synthetase derived from *Saccharomyces cerevisiae* comprising the sequence as shown in SEQ ID NO: 2.

(2) A polypeptide comprising part, and at least a region comprising the N terminus to the residue 237, of the amino acid sequence as shown in SEQ ID NO: 2, which has the activity of the mitochondrial tryptophanyl tRNA

synthetase.

(3) A polypeptide comprising an amino acid sequence derived from the amino acid sequence as shown in SEQ ID NO: 2 by substitution, addition, and/or deletion of 1 or several (for example, 1 to 50, preferably 1 to 30, and more preferably 1 to 10) amino acids and ISTVQ as a region comprising 5 amino acid residues from the N-terminus, which has the activity of the mitochondrial tryptophanyl tRNA synthetase.

(4) A polypeptide comprising ISTVQ as a region comprising 5 amino acid residues from the N-terminus and having 75% or higher, preferably 85% or higher, and more preferably 95% or higher identity with the sequence as shown in SEQ ID NO: 2, which has the activity of the mitochondrial tryptophanyl tRNA synthetase.

(5) A polypeptide comprising the sequence as shown in SEQ ID NO: 1 containing a signal peptide, wherein phenylalanine 38 and/or valine 193 is/are substituted with alanine and/or glycine.

(B) As mt_RNA, various types of mitochondrial suppressor tRNA or $tRNA^{trp}$ can be used. When yeast-derived mtWRS is used, it is preferable to use yeast-derived mt $tRNA^{Trp}$. As the mt $tRNA^{Trp}$ gene, AAGGATATAGTTTAATGGTAAAACAGTTGATTTCAAATCAATCATTAGGAGTTCG AATCTCTTTATCCTTGCCA can be used. Further, in accordance with the preparation of a mutant of yeast cytoplasmic $tRNA^{Trp}$ described in the Examples below, an anticodon site can be mutated from a wild-type to an amber type (CTA), an ochre type (TCA), or an opal type (TTA) (DNA notations). Mutation into an opal type is preferable.

Also, point mutation may be introduced into the acceptor stem or anticodon stem to deteriorate the recognition of aminoacyl tRNA synthetases other than mtWRS, for example. Point mutation can be introduced via PCR using a primer for introducing point mutation.

4. Amino acid analogue

Examples of the non-natural amino acid analogue of the present invention include the following analogues.

Formula 1

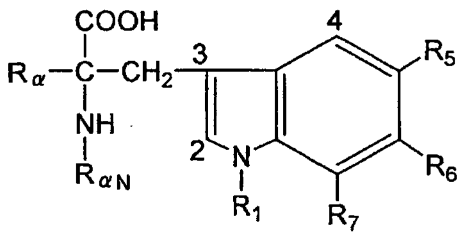

Tryptophan

In Formula 1, Rα, RαN, and $R_1$ each independently represent a branched or unbranched alkyl group having 5 or fewer carbon atoms, with a methyl group being preferable. $R_5$ represents OH, OMe, Me, F, or Br; $R_6$ represents Me or F; and $R_7$ represents Aza or Me. Further, compounds represented by following formulae can also be used:

Formula 2

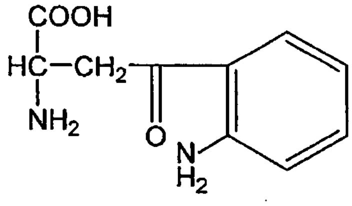

Kynurenine

Formula 3

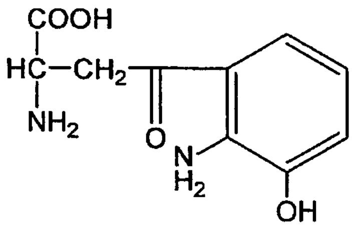

3-Hydroxy Kynurenine

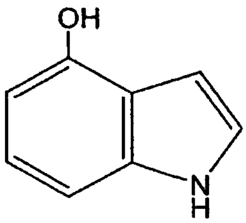

4-Hydroxy indole

Specific examples include alpha-methyl-DL-tryptophan, 1-methyl-DL-tryptophan, 5-methyl-DL-tryptophan, 6-methyl-DL-tryptophan, 7-methyl-DL-tryptophan, 5-fluoro-DL-tryptophan, 5-bromo-DL-tryptophan 6-fluoro-DL-tryptophan, L-abrine, DL-7-azatryptophan, L-kynurenine, 3-hydroxy-DL-kynurenine, 4-hydroxyindole, 5-hydroxy-L-tryptophan, 5-methoxy-DL-tryptophan, and bromo-tryptophan.

By producing non-natural-amino-acid-containing proteins containing such tryptophan analogues, non-natural proteins having the absorption wavelengths and fluorescence wavelengths different from those of natural proteins can be prepared, and target proteins can be assayed or observed while distinguishing them from other proteins. Also, 5-bromo-DL-tryptophan can be suitably used for x-ray crystal structural analysis.

5. Production of non-natural-amino-acid-containing protein

Non-natural proteins comprising non-natural amino acids (amino acid analogues) can be prepared from template mRNA or template DNA, in accordance with the method of the prior patent application by the present inventors (JP Patent Publication (kokai) No. 2004-261160 (A)), using the basic protein synthesis system described in 2. above, using the combination of mtWRS and mt tRNA described in 3. above, and in the presence of the amino acid analogues described in 4. above.

Preferably, the synthesis system contains more tryptophan analogues than natural tryptophan. The amount of mt $tRNA^{Trp}$ or mt $tRNA^{Trp}$ mutants is preferably equivalent to the amount of tRNA that is usually contained in the protein synthesis system.

III. tRNA synthesis

The present invention includes a method of providing an additional sequence to the 5' side of tRNA-encoding DNA to prepare DNA encoding tRNA having an additional sequence on the 5' side, transcribing the DNA encoding tRNA having an additional sequence on the 5' side to prepare precursor tRNA, and treating the precursor tRNA with RNase P to obtain tRNA.

A sequence to be added to the 5' side is any sequence comprising one or several (for example, 1 to 100, preferably 1 to 50, and more preferably 1 to 30) nucleotides, and such sequence comprises G as the first nucleotide. An example is gggagaccacaacggtttccctctaga.

As RNase P used for treating precursor tRNA, RNase P consisting of a C5 protein and M1 RNA or RNase P consisting of M1 RNA can be used.

Examples

In the examples, the following materials were used.

Yeast genomic DNA was prepared from *Saccharomyces cerevisiae* strains (IFO 1234) (Institute for Fermentation). *E. coli* JM109 strains (Promega) and yeast INVSc1 strains (Invitrogen) were used. The pUC19 plasmid (Takara Shuzo Co., Ltd.), the pGEMEX-1 plasmid (Promega), and the pYC2/CT plasmid (Invitrogen) were used. As a cell-free protein synthesis system, a wheat germ extract (the S100 fraction prepared by Ehime University was used and tRNAmix and $^{3}$H-labeled tryptophan were prepared by the laboratory of Hiroyuki Hori, Ehime University) was used. As a medium, LB medium was used (1 liter thereof was prepared by diluting 10 g of Bacto-tryptone (Difco), 5 g of Bacto-yeast extract (Difco), and 10 g of NaCl (Wako) in $dH_2O$). When a plate medium was used, agar (Wako) was added to the above composition to a concentration of 1.5% (w/v). In the case of LB-amp, ampicillin (Bio-101 Inc.) was added to a concentration of 50 μg/ml.

Also, YPD medium (1 liter thereof was prepared by diluting 10g of dried yeast extract (Wako), 20 g of polypepton (Wako), and 20 g of glucose (Wako) in $dH_2O$) was used. As an expression medium, a medium prepared by removing glucose from YPD medium and adding ruffinose (SIGMA-Aldrich) until it became 2% thereof and galactose (Wako) until it became 1% thereof was used. A complete minimal medium was used (selection medium: 1 liter thereof was prepared by diluting 6.7 g of yeast nitrogen base (Difco), 0.1 g of adenine (SIGMA-Aldrich), 0.1 g each of amino acids (arginine, cysteine, leucine, lysine, threonine, tryptophan, aspartate, histidine, isoleucine, methionine, phenylalanine, proline, serine, tyrosine, and valine), and 20 g of glucose in $dH_2O$).

In PCR, TaKaRa Taq™ (Takara Shuzo Co., Ltd.) and Pyrobest DNA polymerase (Takara Shuzo Co., Ltd.) were used.

DNA was purified using EASYTRAP™ (Takara Shuzo Co., Ltd.). Ligation was carried out using Ligation Pack (Nippon Gene Co., Ltd.). Protein purification was carried out using Ni-NTA superflow (QIAGEN) and MagneHis™ Ni-Particles (QIAGEN). As reagents or the like, a protein marker (Daiichi Pure Chemicals Co., Ltd.), Zymolyase-20T (Seikagaku Corporation), ATP, GTP, CTP, and UTP (SIGMA-Aldrich), polyethylene glycol 3400 (ICN Biomedicals Inc.), $^{14}$C-labeled tryptophan (Moravek Biochemical Inc.), and other reagents prepared by Wako were used.

I. Preparation of mitochondrial tryptophanyl tRNA synthetase

[Example 1]

mtWRS cloning

1: Preparation of *E. coli* J109 competent cells

A colony of the E. *coli* JM109 strain was selected using a toothpick, introduced into 2 ml of LB medium contained in a test tube with an aluminum cap, and cultured at 37°C overnight. The cell culture solution (1 ml) was added to 100 ml of LB medium, turbidity (A600) was assayed every 30 minutes, the culture solution was transferred to a 50-ml Falcon tube when the turbidity reached 0.3 to 0.4, the tube was allowed to stand on ice for 15 minutes, centrifugation was carried out using the Beckman Avanti-J25i centrifuge at 6,500 rpm for 15 minutes to harvest the cells, and 4 ml of 1x TSS was added to the cells to carefully suspend such cells therein on ice. The resulting suspension was fractionated in screw cap Eppendorf tubes in amounts of 100 μl each and then used. When competent cells were not to be used immediately, the cells were subjected to liquid nitrogen freezing and then stored at -80°C. When the competent cells stored at -80°C were used, such cells were slowly thawed on ice. 1x TSS was prepared by first preparing 2.5x LB medium, autoclaving the resultant, and aseptically adding 25 ml of 20% (w/v) PEG 6000, 2.5 ml of 1 M $MgCl_2$, and 2.5 ml of DMSO to the solution.

[Example 2]

Preparation of pUCmtWRS

[Example 2-1]

mtWRS gene amplification

The mitochondrial tryptophanyl tRNA synthetase gene (mtWRS gene) was amplified from yeast genomic DNA by PCR (polymerase chain reaction). The sequence ggggggatccaaaaaaatgtctaataagcaggcggttctgaagttaat was used, as the 5' primer and the sequence ggggaccggtctcgaggaagcccattattttatgaatgtcgg was used as the 3' primer for PCR. The composition of the PCR reaction solution and the PCR conditions are shown below. The PCR reaction solution (50 μl)

was prepared by diluting 5 μl of 10x pyrobest buffer, 4 μl of dNTP mix, 1 μl of the 5' primer (100 pmol/μl), 1 μl of the 3' primer (100 pmol/μl), 1 μl of yeast genomic DNA, and 0.5 μl of pyrobest DNA polymerase (5 U/μl) in $dH_2O$ PCR was carried out via a cycle of preliminary denaturation at 95°C for 2 minutes, denaturation at 95°C for 1 minute, annealing at 45°C for 30 seconds, and elongation at 72°C for 10 minutes, and this cycle was repeated 30 times.

To the PCR product, an aqueous solution of 5 M NaCl was added in an amount 1/20 of the PCR product, and the resultant was subjected to ethanol precipitation. The precipitate was dehydrated under reduced pressure to recover a DNA fragment.

The recovered DNA fragment was subjected to 1% agarose gel electrophoresis to confirm that the mtWRS gene had been amplified. The results are shown in Fig. 1. In Fig. 1, M represents a marker (10, 8, 6, 5, 4, 3.5, 3, 2.5, 2, 1.5, 1.2, 1.03, 0.9, 0.8, 0.7, 0.6, 0.5, 0.4, 0.3, 0.2, and 0.1 kbp, from above).

"1" represents a sample after the PCR reaction, and an arrow represents the mtWRS gene of about 1.15 kbp.

According to Fig. 1, a band was detected at a site having substantially the same length as the chain length of the mtWRS gene. Thus, amplification of the mtWRS gene was confirmed.

[Example 2-2]

In order to incorporate the recovered DNA fragment into the pUC19 vector, the pUC19 vector was then digested with HincII. The composition of the reaction solution was as shown below and the reaction was allowed to proceed for 3 hours. A digestion solution (20 μl) was prepared by diluting 2 μl of 10x Y/TANGO™ buffer, 2 μl of pUC19 (0.05 μg/μl), and 1 μl of HincII (10 U/μl) in $dH_2O$.

To the reaction solution after digestion, a 6x loading solution (LS) was added to a concentration of 1x, and the resultant was subjected to 1% agarose gel electrophoresis using 1x TAE buffer and the Gel Mate 2000 (Toyobo) for 30 minutes. The gel was soaked in an ethidium bromide solution, the resultant was observed under UV (355 nm), the relevant band was cleaved out using a razor, and a gel fraction was transferred to an Eppendorf tube.

DNA was extracted from the gel fraction using EASYTRAP™, and the following procedure was carried out in accordance with the protocol. The gel fraction was weighed, an NaI solution was added to the gel fraction in an amount three times that thereof to dissolve the gel fraction at 55°C, 15 μl of glass powder was added thereto, and the resultant was allowed to stand at room temperature for 5 minutes. Thereafter, a microcentrifuge (CFM-100, IWAKI Glass Co., Ltd) was used to perform centrifugation at the maximal speed for 10 seconds, the supernatant was removed, a wash buffer (included in the kit) in an amount 50 times that of the glass powder was added, and the glass powder was thoroughly suspended, followed by washing of the glass powder. This process of washing was repeated twice, 30 μl of $dH_2O$ was added to the precipitated glass powder, and the resultant was heated at 55°C for 10 minutes, followed by extraction of DNA. The resulting solution was centrifuged at the maximal speed for 2 minutes to recover a supernatant, which was a DNA solution. This procedure was repeated twice to purify the DNA fraction. The resultant was subjected to ethanol precipitation, the precipitate was dehydrated under reduced pressure, and the precipitate was dissolved in 30 μl of $dH_2O$. The 6x loading solution contains 0.25% bromophenol blue (BPB), 0.25% xylene cyanol (XC), and 30% (w/v) glycerol. The 1x TAE buffer contains 40 mM Tris-acetic acid (pH.8.3) and 1 mM EDTA.

The DNA solution recovered from the gel fraction was mixed with the PCR product, 0.1 A260 units of yeast tRNA mixture was added as a carrier, and ethanol precipitation was then carried out. The precipitate was dehydrated under reduced pressure, the reaction solution was prepared in the following manner, and the ligation reaction was carried out. The reaction was carried out using the Ligation Pack (Nippon Gene) at 16°C for 2 to 3 hours. The ligation solution (20 μl) was prepared by diluting 2 μl of 10x Ligation buffer, 2.5 μl of the DNA mixture, i.e., the precipitate and BSA (included in the kit), and 0.5 μl of T4 DNA Ligase in $dH_2O$.

After the ligation reaction, the solution was added to 100 μl of the JM109 competent cells, and the mixture was allowed to stand on ice for 5 minutes. In order to perform blue/white selection, the solution was spread on the LB-amp medium plate coated with X-Gal, and the plate was allowed to stand at 37°C overnight. Colonies that had grown white were subjected to the mini-prep method to recover plasmid DNA. This plasmid DNA was designated as pUCmtWRS.

The mini-prep method (the alkali method) was carried out in the following manner. Plasmid DNA was prepared by the alkali method in the following manner. Colonies that had grown on the plate were selected using a toothpick, introduced into 2 ml of LB-amp (50 μg/ml ampicillin) medium in a test tube with an aluminum cap, and then subjected to shaking culture at 37°C overnight. The cell culture solution was fractionated in Eppendorf tubes in amounts of 1.5 ml each, the tubes were subjected to centrifugation at 10,000 rpm for 2 minutes, and the cells were then harvested. As much medium was removed therefrom as possible, the cells were suspended in 100 μl of Solution I, 200 μl of Solution II was added, and the resultant was mildly agitated while shaking the tubes up and down. Subsequently, 150 μl of Solution III was added, the mixture was further agitated, and 150 μl of phenol:chloroform (1:1) solution was added, followed by thorough suspension. The resulting suspension was centrifuged at 12,000 rpm for 10 minutes. The supernatant was recovered and subjected to ethanol precipitation. The precipitate obtained via centrifugation was dissolved in 30 μl of TE buffer. Solution I (TE-glucose buffer) contains 25 mM Tris-HCl (pH 7.6), 10 mM EDTA-Na (pH 7.0), and

50 mM glucose.

Solution II comprises 0.2 M NaOH and 1% SDS, and Solution III (100 ml) comprises 60 ml of 5 M potassium acetate, 11.5 ml of glacial acetic acid, and 28.5 ml of $dH_2O$. TE buffer comprises 10 mM Tris-HCl (pH 8.0) and 1 mM EDTA (pH 8.0).

(Results)

After ligation with pUC19, whether or not the mtWRS gene had been incorporated was confirmed.

The results are shown in Fig. 2. pUCmtWRS was digested with BamHI and with XhoI. The resultant was subjected to 1% agarose gel electrophoresis.

M represents a marker (10, 8, 6, 5, 4, 3.5, 3, 2.5, 2, 1.5, 1.2, 1.03, 0.9, 0.8, 0.7, 0.6, 0.5, 0.4, 0.3, 0.2, and 0.1 kbp, from above).

"1" represents a sample after digestion of pUCmtWRS.

Arrows indicating "1" represent pUC19 (about 2.9 kbp) and mtWRS (about 1.15 kbp), from above.

If pUCmtWRS is digested with BamHI and with XhoI, a pUC19 vector fragment (about 2.9 kbp) and a mtWRS fragment (about 1.15 kbp) should be observed. Since the band was found to be consistent with the prediction, construction of the pUCmtWRS plasmid was confirmed.

[Example 3]

Subcloning into pYC2/CT

The pUCmtWRS precipitate obtained in Example 2-2 and the pYC2/CT vector were digested with BamHI and XhoI restriction enzymes. Digestion was carried out in the same manner as above, and the composition of the reaction solution was as shown below. The digestion solution (20 μl) was prepared by diluting 4 μl of 10x Y/TANGO™ buffer, the pUCmtWRS precipitate, 1 μl of BamHI (10 U/μl), and 1 μl of XhoI (10 U/μl) in $dH_2O$.

The 10x Y/TANGO™ buffer (20 μl) was prepared by diluting 1 μg of pYC2/CT, 1 μl of BamHI (10 U/μl), and 1 μl of XhoI (10 U/μl) in $dH_2O$.

A DNA fragment was purified using agarose gel in the same manner as above; however, a single Eppendorf tube to which the recovered DNA was to be added was used, and ethanol precipitation was carried out. The precipitate was subjected to ligation (using a solution of the same composition as above) and added to the E. *coli* JM109 competent cells to recover pYC2WRS plasmid in the same manner as above.

(Results)

Confirmation of subcloning of mtWRS gene into pYC2/CT vector

If the mtWRS gene was subcloned into the pYC2/CT vector and the resultant was digested with BamHI and with XhoI, a pYC2/CT vector fragment (about 4.5 kbp) and a mtWRS fragment (about 1.15 kbp) should be observed. Thus, pYC2WRS was digested with BamHI and with XhoI and then subjected to 1% agarose gel electrophoresis.

The results are shown in Fig. 3.

M represents a marker (10, 8, 6, 5, 4, 3.5, 3, 2.5, 2, 1.5, 1.2, 1.03, 0.9, 0.8, 0.7, 0.6, 0.5, 0.4, 0.3, 0.2, and 0.1 kbp, from above).

"1" represents a sample after digestion of pYC2WRS.

Arrows indicating "1" represent pYC2/CT (about 4.5 kbp) and mtWRS (about 1.15 kbp), from above. Since the predicted band was actually attained, subcloning of the mtWRS gene into the pYC2/CT vector and construction of the pYC2WRS plasmid were confirmed.

[Example 4]

--mtWRS expression--

(1) Culture of INVSc1 host strain

The INVSc1 host strains for transformation were cultured at 30°C until colonies were grown to a given extent on YPD medium plate. The colonies were precultured in 10 ml of YPD medium at 30°C overnight. YPD medium (50 ml) was prepared to bring the turbidity to 0.4 (OD 600), cells that had been cultured at 30°C for 4 hours were centrifuged at room temperature and 3000x g for 5 minutes, and the cells were then harvested.

(2) Preparation of single-stranded carrier DNA

At the outset, 0.1 g of single-stranded DNA was introduced into a flask, completely dissolved in 10 ml of TE buffer (sterilized), and then allowed to stand at 4°C or higher overnight. Thereafter, DNA was cleaved using an ultrasonic breaker while confirming that DNA fractions were of 7 kbp on average with the aid of agarose gel. The solution was fractionated in centrifugation tubes in amounts of 2.5 ml each, 2.5 ml of TE-saturated phenol was added, centrifugation was carried out at 10,000x g for 5 minutes at 4°C, the supernatant was transferred to the other centrifugation tube, and 2.5 ml of TE-saturated phenol:chloroform (1:1) was added, followed by centrifugation at 10,000x g for 5 minutes at 4°C. Further, the supernatant was transferred to a different centrifugation tube, 2.5 ml of chloroform was added, centrifugation was carried out at 10,000x g for 5 minutes at 4°C, the supernatant was recovered, 3 M sodium acetate in an amount 1/10 that of the supernatant was added, and 12.5 ml of cold ethanol was added to perform ethanol precipitation. The precipitate dehydrated under reduced pressure was added to 10 ml of TE buffer (sterilized), and the resultant was fractionated in Eppendorf tubes, followed by storage at -20°C.

(3) Transformation by the lithium method

The harvested cells were washed with 40 ml of TE buffer. The washed cells were centrifuged, harvested, suspended in 2 ml of 1x LiAc/0.5x TE buffer, and then allowed to stand at room temperature for 10 minutes. This solution (100 μl) was transferred to a screw cap Eppendorf tube, and 1 μg of pYC2WRS and 100 μg of single-stranded carrier DNA were added, followed by thorough pipetting. 1x LiAc/40% PEG-3400/1x TE buffer (700 μl) was added, the resultant was thoroughly mixed, and the mixture was then allowed to stand at 30°C for 30 minutes. Thereafter, 88 μl of DMSO was added, the resultant was thoroughly mixed, and heat shock was carried out at 42°C for 7 minutes. The resultant was centrifuged using a microcentrifuge, and the supernatant was removed. The precipitate was washed with 1 ml of TE buffer, dissolved in 50 μl of TE buffer, and then spread on a complete minimal medium (selection medium) plate. The plate was allowed to stand at 30°C until colonies grew. The 1x LiAc/0.5x TE buffer comprises 100 mM lithium acetate (pH 7.5), 5 mM Tris-HCl (pH 7.5), and 0.5 mM EDTA. The 1x LiAc/40% PEG-3400/lx TE buffer comprises 100 mM lithium acetate (pH 7.5), 40% PEG-3400, 10 mM Tris-HCl (pH 7.5), and 1 mM EDTA.

(4) mtWRS expression

The grown colonies were introduced into 1 liter of selection medium and then subjected to shaking culture at 30°C for 24 hours at 180 rpm. In a clean bench, a preculture solution was then transferred to a 1-1 centrifugation tube that had been sterilized in an autoclave, and the precipitate was recovered using the himac CR22G (Hitachi) at 1,500x g for 5 minutes, followed by suspension in about 200 ml of YPD medium. After the turbidity (OD600) of the suspension was assayed, the suspension was added to 4 liters of expression medium to bring OD600 to 0.4, and the resultant was subjected to shaking culture at 30°C for 24 hours at 180 rpm.

The culture solution was centrifuged in a 1-1 centrifugation tube using himac CR22G at 5,000x g for 3 minutes to recover a precipitate, and the amount of the precipitate was assayed. TSD buffer was added to the cells to a concentration of 0.5 g/ml to suspend the cells therein, and the suspension was allowed to stand at 30°C and 90 rpm for 30 minutes. The suspension was centrifuged at 3,000x g for 5 minutes. The precipitate was suspended in 1.2 M sorbitol and then centrifuged at 3,000x g for 5 minutes. The precipitate was added to and suspended in SKP buffer at a concentration of 0.15 g/ml, Zymolyase 20T was added thereto to a concentration of 5 mg/g, and the reaction was allowed to proceed at 30°C and 90 rpm for about 60 minutes to prepare spheroplasts. The resulting solution was centrifuged at 3,000x g for 5 minutes at 4°C, and the precipitate was resuspended in 1.2 M sorbitol to wash spheroplasts, followed by centrifugation at 3,000x g for 5 minutes at 4°C. This procedure was repeated twice, the precipitate was added to and suspended in MTBP buffer at a concentration of 0.15 g/ml, and the resultant was homogenized with about 10 strokes in a tight-fitting Dounce homogenizer. To the homogenized solution, the same amount of MTBP buffer was added, centrifugation was carried out at 3,000x g for 5 minutes at 4°C, and the supernatant was transferred to a centrifugation tube, followed by centrifugation at 15,000x g for 10 minutes at 4°C. The precipitate was suspended in MTBP buffer, the suspension was centrifuged at 3,000x g for 5 minutes at 4°C, and the supernatant was further centrifuged at 15,000x g for 10 minutes at 4°C. The recovered precipitate was the mitochondrial fraction.

The amount of the precipitate was assayed, the precipitate was suspended in the breaking buffer in an amount twice that of the precipitate, the suspension was transferred to a glass jar (MSK Cell Homogenizer, B. BRAUN), glass beads were added to the same amount of precipitate, and yeast cells were homogenized at 4,000 rpm for 1 minute. The homogenized solution was transferred to a centrifugation tube using Pipetman® while avoiding absorbing glass beads, and the solution was centrifuged using Avanti J25I (Beckman) at 30,000x g for 30 minutes to recover S30 fractions.

mtWRS was purified using an Ni-NTA superflow that would specifically bind to His-tag. At the outset, 250 μl of resin was loaded in Poly-Prep chromatography columns (BIO-RAD) and then equilibrated with 5 ml of breaking buffer.

Subsequently, the supernatant of the homogenized solution was loaded. In order to eliminate nonspecific adsorption, resin was washed with 5 ml of 2x breaking buffer and 5 ml of 2x wash buffer. In order to remove mtWRS that had adsorbed to the Ni-NTA superflow, the solution was eluted using 1 ml of 5x elution buffer at the end. The eluate was concentrated and dialyzed with a storage buffer.

TSD buffer comprises 0.1 M Tris-$SO_4$ (pH 9.4) and 10 mM DTT. SKP buffer comprises 1.2 M sorbitol and 20 mM KPi. MTBP buffer comprises 0.6 M mannitol, 10 mM Tris-HCl (pH 7.4), 0.1% BSA, and 1 mM PMSF. Breaking buffer comprises 50 mM Tris-HCl (pH 7.6), 10 mM $MgCl_2$, 1 mM PMSF (phenyl methyl sulfonyl fluoride), and 5% glycerol. Wash buffer comprises 50 mM Tris-HCl (pH 7.6), 10 mM $MgCl_2$, 5% glycerol, and 10 mM imidazole. Elution buffer comprises 50 mM Tris-HCl (pH 7.6), 10 mM $MgCl_2$, 5% glycerol, and 250 mM imidazole.

Dialysis buffer for storage comprises 20 mM Tris-HCl, 1 mM $MgCl_2$, 6 mM β-mercaptoethanol, 40 mM KCl, and 50% glycerol.

The purity test and the molecular analysis of proteins were carried out via SDS-PAGE. The sample was mixed with the same amount of sample buffer and the mixture was heated at 95°C for 2 minutes. Using SDS buffer, the mixture was electrophoresed at 20 mA until it passed through a concentrate gel, and the current was raised to 40 mA when the mixture entered into a separation gel. After electrophoresis, the product was stained with a Coomassie Brilliant Blue (CBB) R-250 stain, and the product was decolorized with a decoloring solution of CBB staining until a band was detected. The separation gel (8 ml) was prepared by diluting 2 ml of 1.5 M Tris-HCl (pH 8.8), 2 ml of a 40% acrylamide solution (acrylamide:bis = 19:1), and 80 μl of 10% SDS in $dH_2O$. The concentrate gel (4 ml) was prepared by diluting 1 ml of 0.5 M Tris-HCl (pH 6.8), 0.4 ml of a 40% acrylamide solution (acrylamide:bis = 19:1), and 40 μl of 10% SDS in $dH_2O$ SDS sample buffer comprises 0.3 mM Tri-HCl (pH 6.8), 0.7 M β-mercaptoethanol, 0.0013% (w/v) BPB, 2% (w/v) SDS, and 10% (w/v) glycerol. The decoloring solution of CBB staining comprises 25% ethanol and 10% acetic acid. The CBB stain comprises 0.25% CBB-R, 25% ethanol, and 10% acetic acid.

(Results)

mtWRS was expressed using pYC2WRS, the insertion of the mtWRS gene into which had been confirmed. For the purpose of expression confirmation, small amounts of S30 fractions were purified using MagneHis™Ni-Particles, and the resultant was subjected to SDS-PAGE using 10% acrylamide gel. The results are shown in Fig. 4. MagneHis™Ni-Particles were less likely to undergo nonspecific binding, and only one band was observed in elusion. This indicates that mtWRS was expressed. In Fig. 4, M represents a protein marker (molecular weight of 97, 66, 42, and 30 K, from above). Lane 1 indicates a pass-through fraction, lane 2 indicates a wash fraction, and lane 3 indicates an elution (mtWRS) fraction. An arrow indicates a molecular weight of about 41 K.

II. tRNA preparation

[Example 5]

Preparation of C5 protein

The C5 protein gene (rnpA) was amplified from genomic DNA of the E. *coli* JM109 strain by PCR. In order to add the NdeI and XhoI sites, 5'-GGG GCT GCA GCA TAT GGT TAA GCT CGC ATT TCC CAG-3' and 5'-GGG GCT CGA GCC TGG GCG CTC GGT CCG CTG-3' were used as primers (underlined portions indicate NdeI and XhoI sites). The fragment that had been subjected to PCR was cloned into pET21a. The E. *coli* BL21 (DE3) strains transformed with pETC5 were cultured in 1 liter of LB medium until OD600 reached 0.5 to 0.8, and they were induced to express by 0.5 mM IPTG. After the strains were incubated for 4 hours, the expressed cell strains were harvested and frozen. The frozen cells were suspended in 50 ml of 50 mM Tris-HCl (pH 7.6), 5 mM EDTA, 10% glycerol, and 1 M NaCl, and the cells were broken using an ultrasonic breaker (Bioraptor, Tosho Denki, Japan). The cells were centrifuged at 30,000x g for 30 minutes, and the supernatant was then subjected to precipitation with a 50% saturated ammonium sulfate solution. The resultant was centrifuged at 17,000x g for 15 minutes, and the supernatant was then subjected to precipitation with a 80% saturated ammonium sulfate solution. The resultant was centrifuged at 17,000x g for 15 minutes, the recovered precipitate was dissolved in buffer A (50 mM sodium acetate (pH 6.5), 5 mM EDTA, and 0.25 M NaCl), and the resulting solution was further dialyzed against buffer A. The dialyzed protein solution was purified using 10-ml SP Sepharose columns (Amersham). The purified solution was subjected to elution at a flow rate of 2 ml/min with a linear concentration gradient of 0.25-1.0M NaCl (200 ml in total). A C5 protein-containing fraction was recovered, concentrated using Amicon Ultra filters, dialyzed against 20 mM Tris-HCl (pH 7.6), 1 mM $MgCl_2$, 40 mM KCl, 6 mM 2-mercaptoethanol, and 50% glycerol overnight, and then stored at -30°C.

(Results)

C5 proteins were expressed using pETC5, the insertion of the C5 protein gene into which had been confirmed. For the purpose of expression confirmation, small amounts of S30 fractions were purified using Ni-NTA agarose, and the resultant was subjected to SDS-PAGE using 15% acrylamide gel. The results are shown in Fig. 5. Since only one band was observed in elution, expression of C5 proteins was confirmed. In Fig. 5, M represents a protein marker (molecular weight of 14.3 and 18.4 K, from bottom), lane 1 represents an elution fraction (C5 protein), lane 2 represents a wash fraction, lane 3 represents an S30 fraction, and lane 4 represents a pass-through fraction. The right arrow indicates a molecular weight of about 17 K.

[Example 6]

M1 RNA preparation

M1 RNA genome (rnpB) was amplified from genomic DNA of the E. *coli* JM109 strain by PCR. In order to fuse the EcoRI site to the T7 promoter, the primer, 5'-GGG GGA ATT CTA ATA CGA CTC ACT ATA GAA GCT GAC CAG ACA GTC GC-3' (underlined portions), was used. In order to incorporate BamHI, the primer, 5'-GGG GGG ATC CGG ATG ACG TAA ATC AGG TGA AAC TG-3', was used. The fraction after PCR was cloned into the pUC19 plasmid in order to prepare pUCM1. A template for *in vitro* transcription of M1 RNA was amplified from pUCM1 by PCR using a T7 primer that hybridizes to the T7 promoter region (5'-AGG TGA AAC TGA CCG ATA AG-3') and a primer complementary to the 3' terminal region of M1 RNA (5'-AGG TGA AAC TGA CCG ATA AG-3'). *In vitro* transcription was carried out in accordance with Sampson, J. R. and Uhlenbeck, O. C., 1988, Proc. Natl. Acad. Sci., U.S.A., 85, 1033-1037). After transcription, the reaction mixture was purified using a 1-ml Q Sepharose column (Amersham). The purified mixture was subjected to elution at a flow rate of 0.5 ml/min with a linear gradient of 0.4-1.0 M NaCl (20 ml in total). M1 RNA was recovered from the eluate via ethanol precipitation.

(Results)

A template was prepared from pUCM1, the insertion of the M1 RNA gene into which had been confirmed, by PCR amplification. M1 RNA was transcribed and prepared using the resulting template, which was confirmed with the use of 6% acrylamide gel. The results are shown in Fig. 6. Since a band having a length of interest was observed, effective preparation of M1 RNA was confirmed. In Fig. 6, M represents a marker (class 1 tRNA, class 2 tRNA, and 5S rRNA, from bottom), and lane 1 represents a transcript. The right arrow represents M1 RNA (377 nucleotides).

[Example 7]

-- Confirmation of C5 protein and M1 RNA activity--

In order to assay enzyme activity, 150 pmol of the $tRNA^{Tyr}$ precursor having on the 5' end an additional sequence was subjected to the reaction with 30 pmol of C5 proteins and 30 pmol of M1 RNA. The reaction buffer comprises 50 mM Tris-HCl (pH 7.4), 10 mM $MgCl_2$, and 100 mM $NH_4Cl$. The reaction solution was separated using a 7M urea-containing denatured polyacrylamide gel, and nucleic acid components were stained with methylene blue.

(Results)

Each component of RNase P was used to perform the cleavage reaction. The results are shown in Fig. 7. Cleavage activity could not be confirmed with the use of the C5 protein only; however, cleavage activity was confirmed with the use of M1 RNA only. In the presence of the C5 protein and M1 RNA, satisfactory cleavage efficiency was confirmed. In Fig. 7, lane 1 represents before cleavage, lane 2 represents a case where the C5 protein is added, lane 3 represents a case where M1 RNA is added, and lane 4 represents a case both the C5 protein and M1 RNA are added. The right arrows indicate M1 RNA, tRNA having an additional sequence, cleaved functional tRNA, and an additional portion, from above.

[Example 8]

-- Preparation of mitochondrial tRNA$^{Trp}$ and mutant --

(1) Cloning of mt tRNA$^{Trp}$

The gene encoding yeast mitochondrial tRNA$^{Trp}$ (mt tRNA$^{Trp}$) was amplified by PCR. PCR was carried out in the same manner as described above. gggtctagaaaggatatagttta was used as the 5' primer and gggaagcttcctggcaaggataaaga was used as the 3' primer.
The PCR product was purified with 6% polyacrylamide gel in the same manner as described above, digested with HindIII and with XbaI, and then ligated to the pGEMEX-1 vector that had been similarly digested. The product that had been incorporated into pGEMEX-1 was designated as pGEM mtRNA.

(2) Preparation of mt tRNA$^{Trp}$ by transcription

mt tRNA$^{Trp}$ was prepared using pGEM mtRNA as a template by transcription. At the outset, a primer to stop at the 3' terminal CCA was designed in accordance with Kao, C., Zheng, M., and Rudisser, S., 1999, RNA, 5, 1268-1272).
t(Gm)gcaaggataaagagatt was used as the 3' primer, and ggggctgcagtaatacgactcactata was used as the 5' primer (T7 primer).
PCR was carried out using these primers. The composition of the reaction solution and the program are as follows. The PCR reaction solution (50 μl) was prepared by diluting 20 μl of 10x PCR buffer, 16 μl of dNTP mix, 2 μl of the 5' primer (100 pmol/μl), 2 μl of the 3' primer (100 pmol/μl), 4 μl of pGEM mtRNA (diluted to 2,000-fold after the mini-prep method), and 1 μl of Taq DNA polymerase (5 U/μl) in $dH_2O$. PCR was carried out via a cycle of preliminary denaturation of 95°C for 2 minutes, denaturation of 95°C for 30 seconds, annealing of 55°C for 30 seconds, and elongation of 72°C for 30 seconds, and this cycle was repeated 30 times.
After the PCR reaction, 5 μl of the reaction solution was fractionated, 1 μl of LS was added thereto, and amplification was confirmed with the use of 3% GTG agarose gel. To the remaining 195 μl of the reaction solution, the same amount of a phenol:chloroform (1:1) solution was added, the mixture was centrifuged at 15,000 rpm for 10 minutes, the supernatant was introduced into the other Eppendorf tube, and a 5 M NaCl solution in an amount 1/20 of the mixture was added thereto and precipitated with ethanol. The precipitate was dehydrated under reduced pressure and the resultant was used for transcription. The reaction solution (500 μl) was prepared by diluting 50 μl of 10x transcription buffer, 40 μl of 25 mM NTP mix, 10 μl of 1 M $MgCl_2$, the PCR product, the precipitate, and 2 μl of T7 RNA polymerase in $dH_2O$. The 10x transcription buffer comprises 400 mM Tris-HCl (pH 8.0), 10 mM spermidine, 50 mM DTT, and 500 μg/ml BSA.
The obtained mt TRNA$^{Trp}$ contains an extra sequence. Thus, such extra sequence must be cleaved with RNase P in order to obtain active mt tRNA$^{Trp}$. RNase P, which is an enzyme comprising, as the active center, M1 RNA and the C5 protein and prepared in accordance with Examples 5 and 6, was used. M1 RNA and the C5 protein (250 pmol each) were added, and the reaction was allowed to proceed at 37°C for 1 hour. The reaction product was confirmed with the aid of 15% acrylamide urea gel, and purification was carried out using a 2-mm-thick gel plate via gel elution. The total amount of the reaction product was first loaded in 15% acrylamide urea gel, and electrophoresis was performed. The gel was cleaved under UV (355 nm), the gel was transferred to a 15-ml Falcon tube, 4 ml of gel elution buffer was added thereto, and the resultant was subjected to shaking culture at 37°C overnight. On the following day, the solution was recovered, and ethanol precipitation was carried out. To a gel fraction, 2 ml of gel elution buffer was added, the resultant was agitated for about 30 minutes, the reaction solution was mixed therewith, and the resultant was then subjected to ethanol precipitation. The resulting precipitate was dissolved in about 1 ml of $dH_2O$, the gel fraction was removed using the Eppendorf filter, the remnant was transferred to the Eppendorf tube, and ethanol precipitation was performed again to recover mt tRNA$^{Trp}$. The recovered mt TRNA $^{Trp}$ was dissolved with the addition of 100 μl of $dH_2O$, and the concentration was assayed.
The 15% acrylamide urea solution (prepared to 1x TBE) comprises 7M urea, 14.25% acrylamide, and 0.75% N,N'-methylene bisacrylamide.
The gel elution buffer comprises 0.1 M $NH_4COCH_3$, 0.1% SDS, and 1 mM EDTA.

(Results)

After transcription, cleavage reaction was performed using RNase P. The results are shown in Fig. 8. As shown in lane 2, recovery of the target product was confirmed. In Fig. 8, M represents a marker (left arrows represent class 1 tRNA, class 2 tRNA, and 5S rRNA, from bottom), lane 1 represents a product that has not been treated with RNase P, and lane 2 represents a product that has been treated with RNase P. The upper right arrow indicates a product that was

not cleaved by RNase P, and the lower right arrow indicates active tRNA that was cleaved thereby.

[Example 9]

-- Preparation of yeast cytoplasmic $tRNA^{Trp}$ and mutant --

(1) Cloning of cyt $tRNA^{Trp}$ and anticodon mutant

The gene encoding yeast cytoplasmic $TRNA^{Trp}$ (cyt $tRNA^{Trp}$) was amplified by PCR. PCR was carried out in the same manner as described above. gggtctagagaagcggtggctct was used as the 5' primer and gggaagcttcctggtgaaacggacag was used as the 3' primer.

The PCR product was purified with 6% polyacrylamide gel in the same manner as described above, digested with HindIII and with XbaI, and then ligated to the pGEMEX-1 vector that had been similarly digested. The product incorporated into pGEMEX-1 was designated as pGEcyt tRNA. pGEcyt tRNA amber, pGEcyt tRNA ochre, and pGEcyt tRNA opal, the anticodon sites of which had been mutated from the wild types (CCA) to the amber type (CTA), the ochre type (TCA), and the opal type (TTA) (all DNA notations), were prepared. They were prepared by the quick change method involving the use of primers having mutated sequences and PCR.

(2) Preparation of cyt $tRNA^{Trp}$ by transcription

A template was prepared in the same manner as with Example 8 to perform transcription. Thereafter, the transcription product was treated with RNase P to recover the target product.

(Results)

Templates were prepared by PCR amplification from pGEcyt tRNA, pGEcyt tRNA amber, pGEcyt tRNA ochre, and pGEcyt tRNA opal, the insertion of the yeast cytoplasmic $tRNA^{Trp}$ gene and the mutant gene thereof into which had been confirmed, followed by transcription and treatment with RNase P. Preparation of target products was confirmed with the use of 7M urea-containing denatured 10% polyacrylamide gel. The results are shown in Fig. 9. A band having the target length was observed. This indicates that the target product was effectively prepared. In Fig. 9, M represents a marker (right arrows represent class 1 tRNA, class 2 tRNA, and 5S rRNA, from bottom), lane 1 represents wild-type cytoplasmic $tRNA^{Trp}$, lane 2 represents $tRNA^{Trp}$ comprising an amber mutation at the anticodon site, lane 3 represents $tRNA^{Trp}$ comprising an ochre mutation at the anticodon site, and lane 4 represents $tRNA^{Trp}$ comprising an opal mutation at the anticodon site. The upper left arrow represents tRNA that was not cleaved by RNase P, and the lower left arrow represents active tRNA that was cleaved thereby.

Thus, tRNA and the mutant thereof were confirmed to be easily prepared by transcription and with the utilization of RNase P.

[Example 10]

-- Preparation of $tRNA^{Tyr}$ --

pGEMEX plasmid pGEMEX-yTyr was prepared in the following manner. Two oligonucleotides containing complementary regions, i.e., 5'-GGG GTC TAG ACT CTC GGT AGC CAA GTT GGT TTA AGG CGC AAG ACT GTA AAT CTT GAG ATC GGG C-3' and 5'-GGG GAA GCT TGG TCT CCC GGG GGC GAG TCG AAC GCC CGA TCT CAA GAT TT-3', were annealed at 37°C for 1 hour to prepare a double-strand using the Klenow fragment (Takara). The synthetic amber suppressor tRNA gene was digested with XbaI and with HindIII and then ligated to the XbaI and HindIII sites of the pGEMEX-1 (Promega). The sequence was confirmed via dideoxy sequencing. Transcription and *in vitro* processing of tRNA

Yeast $tRNA^{Tyr}$ comprises at the 5' end a pyrimidine ring. Thus, a transcription technique involving the use of T7 RNA polymerase in accordance with Sampson, J. R. and Uhlenbeck, O. C., 1988, Proc. Natl. Acad. Sci., USA, 85, 1033-1037 cannot be employed. Thus, a group of mutant tRNAs were transcribed from the yeast as precursors comprising 27 nucleotides elongated from the 5' region of tRNA (pre-tRNA), and they were then processed with RNase P comprising M1 RNA and the C5 protein. The template for *in vitro* transcription of pre-tRNA was prepared by PCR amplification with the use of the T7 primer and a primer (5'-T (Gm)G TCT CCC GGG GGC GAG T-3'), which is complementary to the 3' end region of the yeast $tRNA^{Tyr}$ and comprises, as the second nucleotide, 2'-O-methylguanosine, in order to refrain from transcriptional elongation, from adequate plasmids comprising relevant genomes cloned into pGEMEX-1 (Kao, C., Zheng, M. and Rudisser, S., 1999, RNA, 5, 1268-1272). Basically, *in vitro* transcription of pre-tRNA was carried out in accordance

with Sampson, J. R. and Uhlenbeck, O. C., 1988, Proc. Natl. Acad. Sci., U.S.A., 85, 1033-1037. pre-tRNA was incubated for 2 hours for transcription, 250 pmol of M1 RNA and the C5 protein were added per ml of the reaction solution, and the reaction mixture was incubated at 42°C for at least 1 hour. The complete tRNA transcript was purified via 7M urea/ 10% PAGE.

(Results)

After the transcription reaction, the product was cleaved with RNase P. The results are shown in Fig. 10. As shown in lane 2, recovery of the target product was confirmed. In Fig. 10, M represents a marker (left arrows represent class 1 tRNA, class 2 tRNA, and 5S rRNA, from bottom), lane 1 represents a product that was not treated with RNase P, and lane 2 represents a product that was treated with RNase P. The upper right arrows represents tRNA that was not cleaved by RNase P, a middle right arrow represents active tRNA that was cleaved thereby, and the lower right arrow represents a cleaved additional sequence.

III. Analysis of MtWRS

[Example 11]

-- Analysis of mt WRS --

Assay of aminoacylation activity

In order to assay enzyme activity, aminoacylation was carried out using $^{14}C$ tryptophan. The reaction solution having the composition shown below was heated at 30°C for about 5 minutes while the enzyme was removed therefrom. Thereafter, the enzyme was added, the mixture was incubated at 30°C, the mixture was spotted on a filter wetted with 5% TCA 0, 1, 4, 9, and 16 minutes after incubation, and the filter was then soaked in 5% TCA. After spotting of the reaction solution at each time point was completed, 5% TCA was discarded, and fresh 5% TCA was added to the beaker, followed by shaking for 10 minutes. This procedure was repeated twice, 100% ethanol was added, and the mixture was lightly shaken, followed by dehydration under an electric heat. Thereafter, the resultant was soaked in a scintillator, and the samples were counted using a liquid scintillation counter.

The reaction solution for aminoacylation (50 μl) was prepared by diluting 10 μl of 5x AAM (aminoacylation mixture), 0.05 A260 units of mt $tRNA^{Trp}$, an adequate amount of enzyme (depending on the concentration), and 2 μl of $^{14}C$ tryptophan in $dH_2O$.

The 5x AAM (aminoacylation mixture) comprised 500 mM Tris-HCl (pH 7.6), 50 mM $MgCl_2$, 200 mM KCl, and 20 mM ATP.

A scintillator (1 liter) was prepared by diluting 4 g of Dotite DPO and 0.1 g of Dotite POPOP in toluene.

(Results)

The capacity of tRNA for accepting $^{14}C$-tryptophan was confirmed using mtWRS and mt $tRNA^{Trp}$. The results are shown in Fig. 11. The calculation formulae are shown below.

Based on the $^{14}C$ tryptophan radioactivity of 48 mCi/mmol (1 mCi = 37 MBq),
37 MBq x 48 mCi/mmol = 1776 MBq/mmol = 1.8 GBq/mmol
Based on 1 dpm = 60 dps and 1 Bq = 1 dps,
1.8 GBq/mmol = 1.8 Bq/pmol = 1.8 dps/pmol x 60 sec = 108 dpm/pmol Since the amount of the sample to be spotted on the filter is 9 μl out of 50 μl, the tRNA amount per spot is

$$0.05 \times 9/50 = 0.009 \text{ A260 units.}$$

Thus, the capacity for acceptance (pmol/A260 unit) = measured value/(108 x 0.009).

Although the concentration of the enzyme added was somewhat high, acceptance of tryptophan was observed. Thus, the expressed substance was confirmed to be mtWRS. Since acceptance was not observed when either of the enzyme or tRNA was absent, acceptance was found to occur only when both the enzyme and tRNA were added.

[Example 12]

Identification of signal peptide cleavage site of mtWRS

A sample for peptide sequencing was prepared. The method thereof is described below. A separation gel and a concentrate gel were prepared for SDS-PAGE, and electrophoresis was carried out using an anolyte and a catholyte. The concentrate gel was cleaved, the cleaved gel was soaked in E. blotting buffer C for 5 minutes, a filter was cut in accordance with the gel size, and the filter was soaked in the buffer. A PVDF membrane was soaked in methanol and then in E. blotting buffer C for 5 minutes. The buffer, the gel, and the membrane were aligned as shown in Fig. 12, and a current was applied at 1 $mA/cm^2$ (c.c.) for 90 minutes. After electroblotting, the PVDF membrane was washed with $dH_2O$ and then stained with Ponceau S. The membrane was decolored using a decoloring solution (1% AcOH) and washed with $dH_2O$. A band was cleaved, introduced into the Eppendorf tube, and stored at 4°C until peptide sequencing was performed.

The anolyte (100 ml) was prepared by diluting 24.2 g of Tris-HCl (pH 8.9) in $dH_2O$.

The catholyte (1 liter) was prepared by diluting 12.1 g of Tris, 17.9 g of Tricine, and 1.0 g of SDS in $dH_2O$. The E. blotting buffer A (1 liter) was prepared by diluting 36.3 g of Tris, 5 ml of 10% SDS, and 200 ml of methanol in $dH_2O$. The 2x E. blotting buffer B (500 ml) was prepared by diluting 3 g of Tris, 5 ml of 10% SDS, and 200 ml of methanol in $dH_2O$. The E. blotting buffer C (1 liter) was prepared by diluting 500 ml of 2x E. blotting buffer B and 5.2 g of 6-amino-N-caproic acid in $dH_2O$.

Peptide sequencing was carried out using the Peptide Sequencer (ABI) to read 5 residues at the N terminus.

(Results)

Identification of signal peptide cleavage site

In order to identify the signal peptide cleavage site, the N-terminal amino acid sequence was inspected using a peptide sequencer. The results (Table 1) and the identified sequences are shown below.

Table 1

| N-terminal sequence/Call | First | Second | Third |
|---|---|---|---|
| 1 | Ile | Lys | Asp |
| 2 | Ser | Asp | Arg |
| 3 | Thr | Leu | Lys |
| 4 | Val | Arg | Asn |
| 5 | Gln | Glu | Ala |
| Identified N-terminal sequence comprising 5 amino acids: ISTVQ | | | |

[Example 13]

Confirmation of cross reaction between wheat germ extract-derived TrpRS and tRNA

Whether or not the pair of mtWRS and mt $tRNA^{Trp}$ could be used as the pair for introducing a non-natural amino acid in the wheat germ extract was examined via cross-reaction via aminoacylation.

At the outset, S100 fractions were prepared from wheat germ in the following manner. Wheat germ (1 g) was wrapped in a gauze and washed with MilliQ in a beaker. Further, the resultant was thoroughly washed with 0.5% Nonidet P40 in an ultrasonic washer, followed by substitution with $dH_2O$, to perform ultrasonic washing. The washed germ was sandwiched between filters for dehydration, and the resultant was introduced into a mortar, which had been cooled to -80°C, followed by breaking with the addition of liquid nitrogen. The broken germ was transferred to the other mortar, 5 ml of buffer A was added for pasting, and the resultant was subjected to ultracentrifugation at 100,000x g for 2 hours at 4°C. The supernatant was recovered and loaded in Poly-Prep chromatography columns, into which 3 ml of DE52 resin had been filled.

(Nucleic acid removal)

The eluate obtained with buffer B was dialyzed against a dialysis buffer overnight. The resultant was designated as the S100 fraction. Buffer A (100 ml) was prepared by diluting 50 mM Tris-HCl, 5 mM $MgCl_2$, 1 mM DTT, and 50 mM KCl in $dH_2O$. Buffer B (50 ml) was prepared by diluting 50 mM Tris-HCl, 5 mM $MgCl_2$, 1 mM DTT, and 300 mM KCl in $dH_2O$. The dialysis buffer (500 ml) was prepared by diluting 50 mM Tris-HCl, 5 mM $MgCl_2$, 1 mM DTT, 50 mM KCl, and 50% glycerol in $dH_2O$.

The S100 fraction and wheat tRNA mix were used to confirm the cross reaction by aminoacylation. Aminoacylation was carried out using the solution of the following composition. The pair of wheat germ S100 and wheat tRNA mix (50 μl) was prepared by diluting 10 μl of 5x AAM, 10 μl of S100, 2 A260 units of tRNA mix, and 3 μl of $^3$H-tryptophan in $dH_2O$. The pair of wheat germ S100 and mt $tRNA^{Trp}$ (50 μl) was prepared by diluting 10 μl of 5x AAM, 10 μl of S 100, 0.5 A260 units of mt $tRNA^{Trp}$, and 3 μl of $^3$H-tryptophan in $dH_2O$. The pair of wheat germ S100 and wheat tRNA mix (50 μl) was prepared by diluting 10 μl of 5x AAM, 0.5 μg of mtWRS, 2 A260 units of tRNA mix, and 3 μl of $^3$H-tryptophan in $dH_2O$. The pair of mtWRS and mt $tRNA^{Trp}$ (50 μl) was prepared by diluting 10 μl of 5x AAM, 0.5 μg of mtWRS, 0.5 A260 units of mt $tRNA^{Trp}$, and 3 μl of $^3$H-tryptophan in $dH_2O$.

(Results)

The wheat germ extract S100 fraction (without nucleic acid) and the wheat tRNA mix were used to confirm the cross-reaction between mtWRS and mt $tRNA^{Trp}$. The results are shown in Fig. 13.

In Fig. 13, x represents the pair of mtWRS and mt $tRNA^{Trp}$, ♦ (slanted square) represents the pair of the wheat S 100 fraction (without nucleic acid) and the wheat tRNA mix, ■ (square) represents the pair of the wheat S 100 fraction (without nucleic acid) and mt $tRNA^{Trp}$, and ▲ (triangle) represents the pair of mtWRS and wheat tRNA mix.

Based on the results, the pair of mtWRS and mt $tRNA^{Trp}$ and the pair of WRS and tRNA in wheat germ were found to cause aminoacylation. Thus, the pair of mtWRS and mt $TRNA^{Trp}$ was found to cause no cross reaction in the wheat germ extract.

[Example 14] Opal codon suppression

(1) Preparation of pEUDHFRopal

With the use of commercialized pEU-DHFR (Toyobo), the following primers were designed in order to mutate tyrosine 128 into an opal stop codon, which is known to be unrelated to activity.
Primer F: T TTC CCG GAT TGA GAGCCG GAT G
Primer R: C ATC CGG CTC TCA ATC CGGG AAA

With the use of these two primers, position 128 of pEU-DHFR for the wheat germ extract-derived cell-free protein synthesis system was mutated into an opal codon by PCR under the following reaction conditions.

The reaction solution (50 μl) used was prepared by diluting 5 μl of 10x pyrobest buffer, 4 μl of dNTP mix, 1 μl of primer F (100 pmol/μl), 1 μl of primer R (100 pmol/μl), 0.1 μg of pEU-DHFR, and 0.5 μl of pyrobest DNA polymerase (5U/μl) in $dH_2O$.

PCR was carried out via a cycle of preliminary denaturation at 95°C for 5 minutes, denaturation at 95°C for 1 minute, annealing at 50°C for 30 seconds, and elongation at 72°C for 12 minutes, and this cycle was repeated 18 times.

The PCR product was subjected to ethanol precipitation, dissolved in 10 μl of $dH_2O$, added to the XL1 1 Blue competent cells for transformation, spread on an LB-amp plate, and then cultured overnight. The grown colonies were introduced into 2 ml of LB-amp liquid medium in a test tube with an aluminum cap and cultured overnight.

On the following day, liquid medium was introduced into an Eppendorf tube and plasmids were recovered by the alkali method. The resultant was inspected by DNA sequencing to confirm that position 128 had been mutated into TGA. The obtained plasmid was designated as pEUDHFRopal.

Subsequently, mRNA was prepared via *in vitro* transcription under the following reaction conditions in order to use such mRNA for the wheat germ extract-derived cell-free protein synthesis system.

(2) Inspection of opal codon suppression using the wheat germ extract-derived cell-free protein synthesis system

With the use of the obtained mRNA, an opal codon was subjected to suppression using the wheat germ extract-derived cell-free protein synthesis system, i.e., PROTEIOS™ (Toyobo).

Specifically, the reaction solutions having the following compositions were used.

(i) DHFR-WT (12.5 μl, a control reaction solution using wild-type DHFR-encoding mRNA) was prepared by diluting

0.85 μl of Buffer #1 (included in the kit), 1.5 μl of Buffer #2 (included in the kit), 0.5 μl of creatine kinase (10 mg/ml), 0.5 μl of $^{14}C$-leucine, 3 μg of mRNA (WT), 0.25 μl of RNase inhibitor (40 U/μl), and 2.5 μl of wheat germ extract in $dH_2O$.

(ii) DHFRopal+mtWRS (12.5 μl, a reaction solution prepared by adding mtWRS to mRNA encoding DHFR into which the opal mutation had been introduced) was prepared by diluting 0.85 μl of Buffer #1, 1.5 μl of Buffer #2, 0.5 μl of creatine kinase (10 mg/ml), 0.5 μl of $^{14}C$-leucine, 3 μg of mRNA (WT), 0.25 μl of RNase inhibitor (40 U/μl), 2.5 μl of wheat germ extract, and 1.2 μg of mtWRS in $dH_2O$.

(iii) DHFRopal+mtWRS+mt $tRNA^{Trp}$(12.5 μl, a reaction solution prepared by adding mtWRS and mt $tRNA^{Trp}$ to mRNA encoding DHFR into which the opal mutation had been introduced) was prepared by diluting 0.85 μl of Buffer #1 (included in the kit), 1.5 μl of Buffer #2 (included in the kit), 0.5 μl of creatine kinase (10 mg/ml), 0.5 μl of $^{14}C$-leucine, 3 μg of mRNA (WT), 0.25 μl of RNase inhibitor (40 U/μl), 2.5 μl of wheat germ extract, 1.2 μg of mtWRS, and 0.05 A260 units of mt $tRNA^{Trp}$ in $dH_2O$.

Protein synthesis was carried out using the above reaction solutions at 28°C for 2 hours. Acetone (100 μl) was added to the reaction solution, the mixture was allowed to stand at -30°C for 5 minutes, and the resultant was centrifuged at 15,000 rpm for 10 minutes. This procedure was repeated twice and the resultant was then dehydrated. The product was subjected to 20% SDS-PAGE, and the electrophoresed product was sandwiched between a film and a filter to dehydrate a gel, followed by detection with the use of an imaging plate.

(Results)

The results are shown in Fig. 14. In general, a peptide fragment that had been terminated at UGA is solely synthesized. With the addition of novel tRNA/mtWRS, a full-length dihydrofolate reductase, which had read through UGA as tryptophan, is synthesized, as is apparent from Fig. 14.

[Example 15] Modification of yeast mitochondrial tryptophanyl tRNA synthetase (mtWRS)

(1) Preparation of mtWRS mutant F38A gene

In order to have mt $tRNA^{Trp}$ to accept non-natural amino acids, amino acid specificity of mtWRS was modified.

With reference to the B. stearothermophilus WRS, the crystal structure of which had been reported, mtWRS was first compared with amino acid sequences of various WRS to deduce a tryptophan-binding site. Among such various types of mtWRS, phenylalanine 38 of the mtWRS that is considered to be involved with immobilization of an indole ring on tryptophan was mutated into alanine or glycine to design primer DNA (Fig. 15).

Primer 1: T GCT ACG GTA GSC AGT ATG ATT C

Primer 2: G AAT CAT ACT GSC TAC CGT AGC A S=G or C

With the use of these 2 primers, position 38 of the pYCmtWRS plasmid for mtWRA yeast expression was modified into alanine by PCR using the following reaction solution under the following reaction conditions.

The reaction solution (50 μl) was prepared by diluting 5 μl of 10x pyrobest buffer, 4 μl of dNTP mix, 1 μl of primer 1 (100 pmol/μl), 1 μl of primer 2 (100 pmol/μl), 0.1 μg of pYCmtWRS, and 0.5 μl of pyrobest DNA polymerase (5 U/ μl) in $dH_2O$.

PCR was carried out via a cycle of preliminary denaturation at 95°C for 5 minutes, denaturation at 95°C for 1 minute, annealing at 50°C for 30 seconds, and elongation at 72°C for 12 minutes, and this cycle was repeated 18 times.

The PCR product was subjected to ethanol precipitation, dissolved in 10 μl of $dH_2O$, added to the XL 1 Blue competent cells for transformation, spread on an LB-amp plate, and then cultured overnight. The grown colonies were introduced into 2 ml of LB-amp liquid medium in a test tube with an aluminum cap and cultured overnight.

On the following day, liquid medium was introduced into an Eppendorf tube and plasmids were recovered by the alkali method. The resultant was inspected by DNA sequencing to confirm that position 38 had been mutated into alanine. The obtained plasmid was designated as pYC38A.

(2) Expression and purification of F38A

The host INVSc strains were subjected to streak culture on YPD medium plate, and colonies were allowed to grow to a given size at 30°C. The grown colonies were cultured using 10 ml of YPD medium at 30°C overnight. On the following day, YPD medium was added to the culture solution to bring the turbidity to OD600 of 0.4, culture was conducted at 30°C for 4 hours, and centrifugation was carried out at 3,000x g for 5 minutes to harvest cells in a Falcon tube.

The cells were then washed with 40 ml of TE buffer (sterilized). The cells were recovered by centrifugation, suspended in 2 ml of 1x LiAc/0.5x TE buffer (sterilized), and then allowed to stand at room temperature for 10 minutes.

The resulting solution (100 μl) was transferred to a screw cap Eppendorf tube, and 1 μg of pYC38A and 100 μg of carrier DNA were added and thoroughly suspended therein. To the resulting solution, 700 μl of 1x LiAc/40% PEG3400 was added, and the mixture was then allowed to stand at 30°C for 30 minutes. Subsequently, 88 μl of DMSO was added, and heat shock was imparted at 42°C for 7 minutes. The solution was centrifuged, the supernatant was removed, and the precipitate was then washed with 1 ml of TE buffer (sterilized). After centrifugation, the product was suspended in an adequate amount of TE buffer (sterilized) and spread on a selection medium plate.

The grown colonies were inoculated in 100 ml of selection medium (glucose) and cultured at 30°C overnight. The culture solution was added to 1 liter of selection medium (glucose) and culture was conducted at 30°C overnight. The resulting culture solution was introduced into a sterilized centrifugation tube to harvest cells, the harvested cells were suspended in 1 liter of selection medium (raffinose), and culture was then conducted at 30°C for 1 hour. The resulting culture solution was transferred to 10 liters of YPR +1% galactose medium to perform expression culture for 24 hours.

YPD medium and YPR medium (1 liter each) were prepared by diluting 10 g of yeast extract, 20 g of polypepton, and 10 g of glucose or raffinose in $dH_2O$. TE buffer was composed of 10 mM Tris-HCl (pH 7.5) and 1 mM EDTA.

Cells were recovered, the recovered cells were added to and suspended in TSD buffer (comprising O.1M Tris-$SO_4$ (pH 9.4) and 10 mM DTT) to a concentration of 0.5 g/ml, and the resultant was allowed to stand at 30°C for 10 minutes. The cells were centrifuged at 3,000x g for 5 minutes, and the precipitate was washed with 1.2 M sorbitol. Subsequently, the cells that had been centrifuged at 3,000x g for 5 minutes were added to and suspended in SKP buffer (comprising 1.2 M sorbitol and 20 mM KPi (pH 7.6)) to a concentration of 0.15g/ml, 5 mg of Zymolyase 20T (Seikagaku Corporation) was added thereto per g of the cells, and the mixture was allowed to react at 30°C for 3 hours to prepare spheroplasts. The resulting suspension was centrifuged at 3,000x g for 5 minutes, and the precipitate was washed three times with 1.2 M sorbitol. After centrifugation, MTBP buffer (comprising 0.6 M mannitol, 10mM Tris-HCl (pH7.4), 0.1% BSA, and 1mM PMSF) was added to the spheroplasts to a concentration of 0.15g/ml, and the cells were broken using a tight-fitting Dounce homogenizer. The solution of the broken cells was centrifuged at 3,000x g for 5 minutes, and the supernatant was centrifuged at 15,000x g for an additional 10 minutes. The resulting precipitate was recovered as a mitochondrial fraction.

The precipitate was suspended in 10 ml of breaking buffer and ultrasonically broken for 30 minutes. The resulting solution was centrifuged at 30,000x g for 30 minutes to recover S30 fractions. Ni-NTA superflow (500 μl, QIAGEN) was filled in the Poly-Prep chromatography columns (BIO-RAD), and resin was equilibrated with 5 ml of breaking buffer. The S30 fraction was loaded therein to bind F38A to the resin. The resin was washed three times with a wash buffer, and F38A was then eluted 4 times with 500 μl of elution buffer. The breaking buffer comprised 50 mM Tris-HCl (pH 7.6), 10 mM $MgCl_2$, 1 mM PMSF (phenyl methyl sulfonyl fluoride), and 5% glycerol. The wash buffer comprised 50 mM Tris-HCl (pH 7.6), 10 mM $MgCl_2$, 5% glycerol, and 10 mM imidazole. The elution buffer comprised 50 mM Tris-HCl (pH 7.6), 10 mM $MgCl_2$, 5% glycerol, and 250 mM imidazole.

Subsequently, elution was performed with the use of cation-exchange columns, HiTrap™SP HP (1 ml, Amersham Bioscience), and a buffer containing 20 mM PIPES-KOH (pH 6.6), 1 mM $MgCl_2$, and 5% glycerol, at a flow rate of 0.5 ml/min, with a linear concentration gradient of 0 to 0.5 M KCl (30 ml in total).

The vicinity of the fraction, the peak was observed via SDS-PAGE, was inspected. The resulting F38A was concentrated using Amicon Ultra filters (Millipore) and dialyzed against a dialysis buffer (20 mM Tris-HCl (pH 7.6), 1 mM $MgCl_2$, 40 mM KCl, and 50% glycerol) overnight.

(3) Confirmation of amino acid specificity of F38A

The acceptance of tryptophan and that of an tryptophan analogue to mt $tRNA^{Trp}$ by mtWRS and F38A were confirmed via formation of a triple complex using EF-Tu of *T. Thermophilus.* EF-Tu forms a complex with EF-Ts. Upon contact of GTP with this complex, EF-Ts is dissociated. In the presence of aminoacyl tRNA, a complex of aminoacyl tRNA/EF-Tu/GTP is formed. That is, a triple complex is formed when tryptophan and an tryptophan analogue are accepted by mt $tRNA^{Trp}$.

The reaction was carried out in the same manner concerning each tryptophan and the tryptophan analogue under the following conditions.

The reaction solution was composed of 50 mM Tris-HCl (pH 7.6), 50 mM KCl, 50 mM $NH_4Cl$, 7 mM $MgCl_2$, 5 mM β-mercaptoethanol, 2 mM ATP, 1 mM GTP, 0.1 mM EF-Tu, 0.01 mM EF-Ts, 0.003 A260 units/μl of mt $tRNA^{Trp}$, 0.05g/L mtWRS or F38A, and 0.1 mM tryptophan or a tryptophan analogue.

The reaction was allowed to proceed using such reaction solution at 37°C for 30 minutes, and 6% TAM-PAGE was then carried out for 1 hour. The electrophoresed gel was stained with CBB-R, and the decolored gel was stained with methylene blue to detect a band.

6% TAM-PAGE was carried out using 10 ml of a solution prepared by diluting 0.5 ml of 20x TAM and 1.5 ml of a 40% acrylamide solution in $dH_2O$. 20x TAM was 0.5M Tris base and it comprised 0.5 M acetic acid and 0.1 M Mg

$(OAc)_2$.

(Results)

The results are shown in Fig. 16, and the results of analysis thereof are shown in Fig. 17.
The results attained in Examples 14 and 15 demonstrate that utilization of the F38A mutant and the wheat germ extract-derived cell-free protein synthesis system enables the introduction of an tryptophan analogue in a UGA-specific manner.

Industrial Applicability

According to the present invention, a non-natural amino acid protein comprising a mutant amino acid in a site-specific manner can be assuredly and effectively produced. Further, a non-natural amino acid protein comprising an tryptophan analogue into which effective introduction of an analogue has been heretofore expected can be produced.
Also, use of the novel mitochondrial tryptophanyl tRNA synthetase of the present invention enables development of novel antibiotics and pharmaceutical products.
Further, the present invention enables the preparation of tRNA comprising a sequence having, as the first nucleotide, a nucleotide other than G. This in turn facilitates the production of a mutant thereof.
All publications, patents, and patent applications cited herein are incorporated herein by reference in their entirety.

## Claims

1. A method for producing a non-natural-amino-acid-containing protein comprising an amino acid at a given site of the protein mutated into a non-natural amino acid, comprising adding a mutant gene in which an amino acid codon at a given site of the protein-encoding gene has been mutated into an opal codon, a mitochondrial tryptophanyl tRNA synthetase (mtWRS), and mitochondria $tRNA^{Trp}$ (mt $tRNA^{Trp}$) or an opal suppressor tRNA to a protein translation/ synthesis system.

2. The method according to claim 1, wherein the protein synthesis system is a cell-free protein synthesis system or an eukaryotic cell.

3. The method according to claim 2, wherein the cell-free protein synthesis system is a wheat germ-derived cell-free protein synthesis system.

4. The method according to any one of claims 1 to 3, wherein the mitochondrial tryptophanyl tRNA synthetase (mtWRS) and mitochondrial $TRNA^{Trp}$ (mt $tRNA^{Trp}$) are yeast mitochondrial tryptophanyl tRNA synthetase (mtWRS) and yeast mitochondrial $tRNA^{Trp}$ (mt $tRNA^{Trp}$).

5. The method according to any one of claims 1 to 4, wherein the non-natural amino acid is a tryptophan analogue.

6. A mitochondrial tryptophanyl tRNA synthetase comprising any of the following polypeptide (1), (2), or (3):

(1) a polypeptide comprising the amino acid sequence as shown in SEQ ID NO: 2;
(2) a polypeptide comprising part, and at least a region comprising the N terminus to the residue 237, of the amino acid sequence as shown in SEQ ID NO: 2; or
(3) a polypeptide comprising an amino acid sequence derived from the amino acid sequence as shown in SEQ ID NO: 2 by substitution, addition, and/or deletion of 1 or several amino acids and ISTVQ as a region comprising 5 amino acid residues from the N-terminus, which has the activity of the mitochondrial tryptophanyl tRNA synthetase.

7. A nucleic acid as shown in SEQ ID NO: 4 or a nucleic acid that hybridizes under stringent conditions to a nucleic acid complementary to the nucleic acid as shown in SEQ ID NO: 4 and that encodes a polypeptide having the activity of the tryptophanyl tRNA synthetase.

8. A method for producing a yeast mitochondrial tryptophanyl tRNA synthetase comprising introducing a recombinant expression vector comprising the gene having the sequence as shown in SEQ ID NO: 4 into *E. coli.*

9. An antibody that specifically reacts with the mitochondrial tryptophanyl tRNA synthetase according to claim 6.

10. A method for screening for a tRNA synthetase inhibitor comprising inspecting the capacity of tRNA for accepting tryptophan in a solution containing the mitochondrial tryptophanyl tRNA synthetase and mitochondrial $tRNA^{Trp}$ (mt $tRNA^{Trp}$), in the presence of a candidate inhibitor against the mitochondrial tryptophanyl tRNA synthetase according to claim 6.

11. A method for synthesizing tRNA comprising the following steps of:

(1) providing an additional sequence to the 5' side of tRNA-encoding DNA to prepare DNA encoding tRNA having an additional sequence on the 5' side;
(2) transcribing the DNA encoding tRNA having an additional sequence on the 5' side to prepare precursor tRNA; and
(3) treating the precursor tRNA with RNase P to obtain tRNA.

12. The method according to claim 11, wherein tRNA is mt $tRNA^{Trp}$.

13. The method according to claim 11, wherein the additional sequence on the 5' side is gggagaccacaacggtttccctctaga.

14. The method according to claim 11, wherein the RNase P comprises a C5 protein and M1 RNA.

**15.** The method according to claim 11, wherein the RNase P comprises M1 RNA.

**16.** The method according to claim 1, which involves the use of tRNA prepared by the method according to any of claims 11 to 15.

Fig. 1

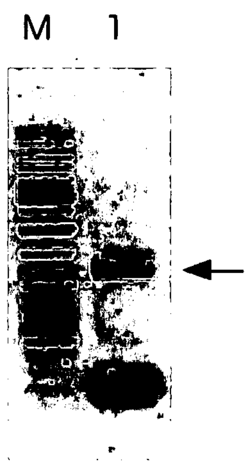

Fig. 2

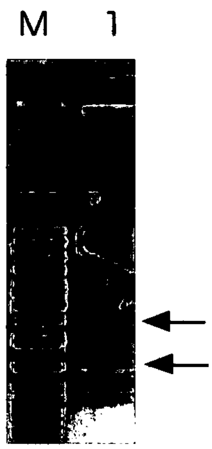

Fig. 3

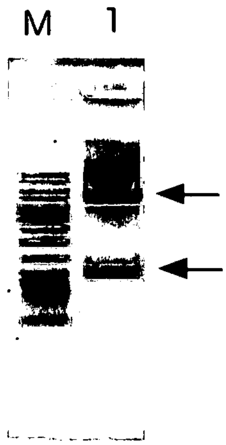

Fig. 4

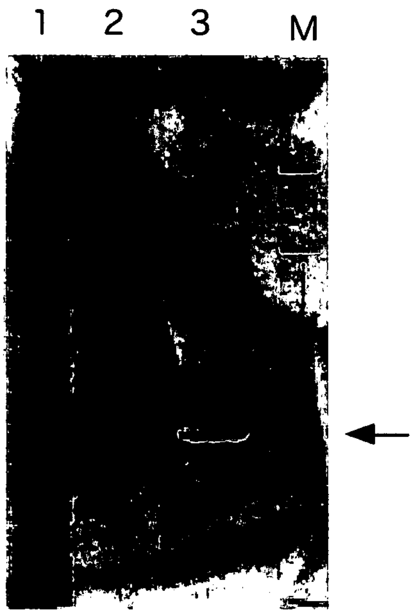

Fig. 5

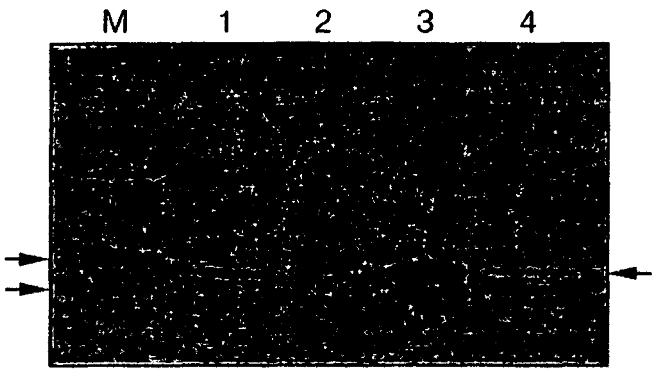

Fig. 6

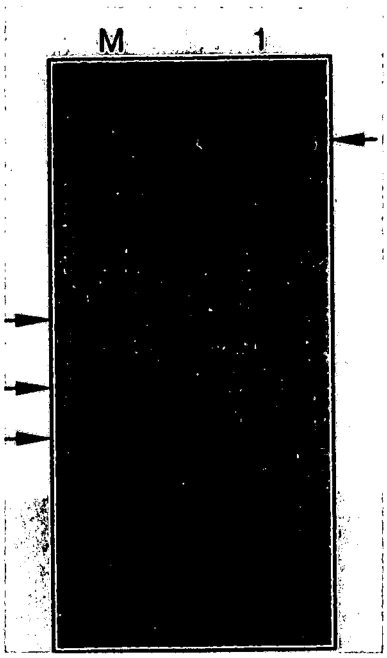

Fig. 7

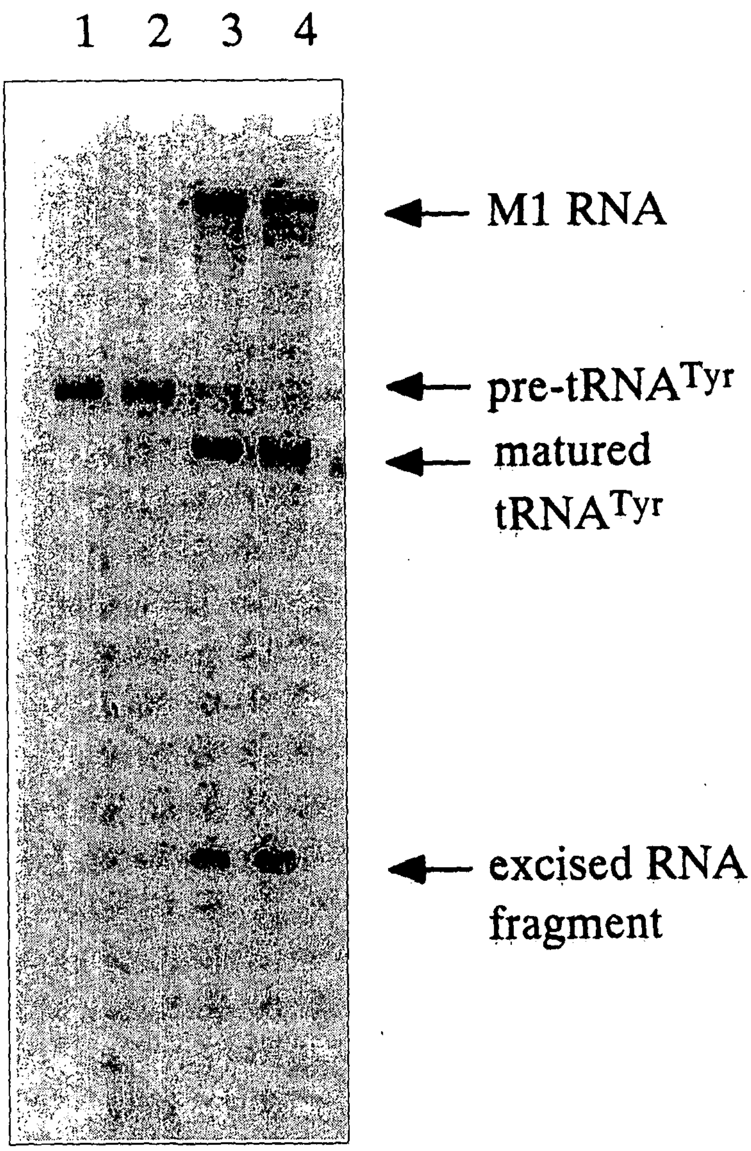
1 2 3 4
M1 RNA
pre-tRNATyr
matured tRNATyr
excised RNA fragment

Fig. 8

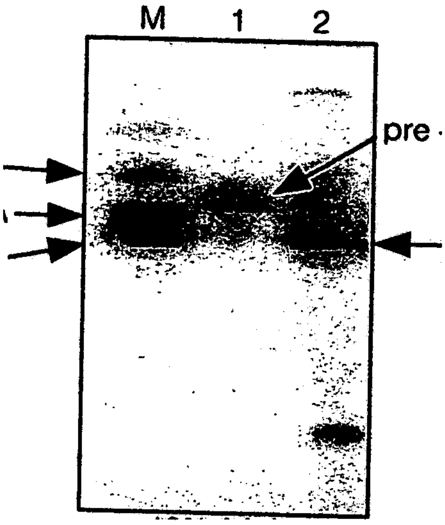

Fig. 9

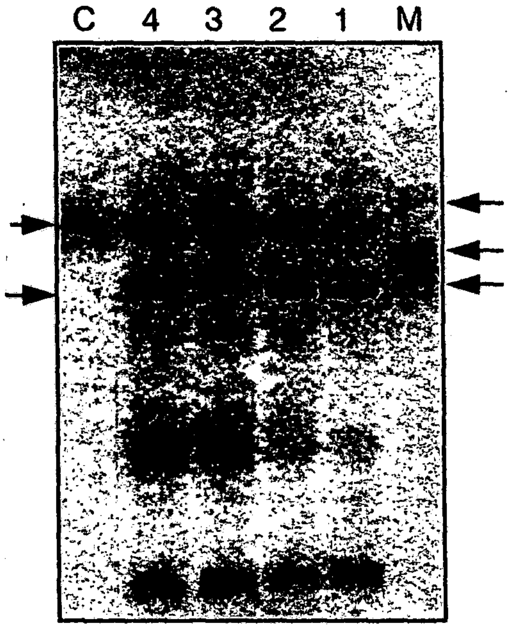

Fig. 10

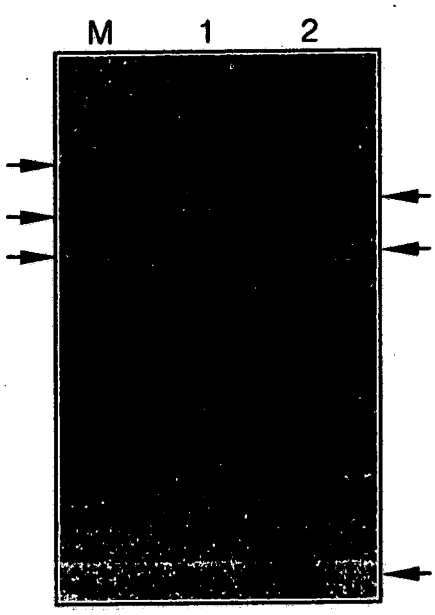

Fig. 11

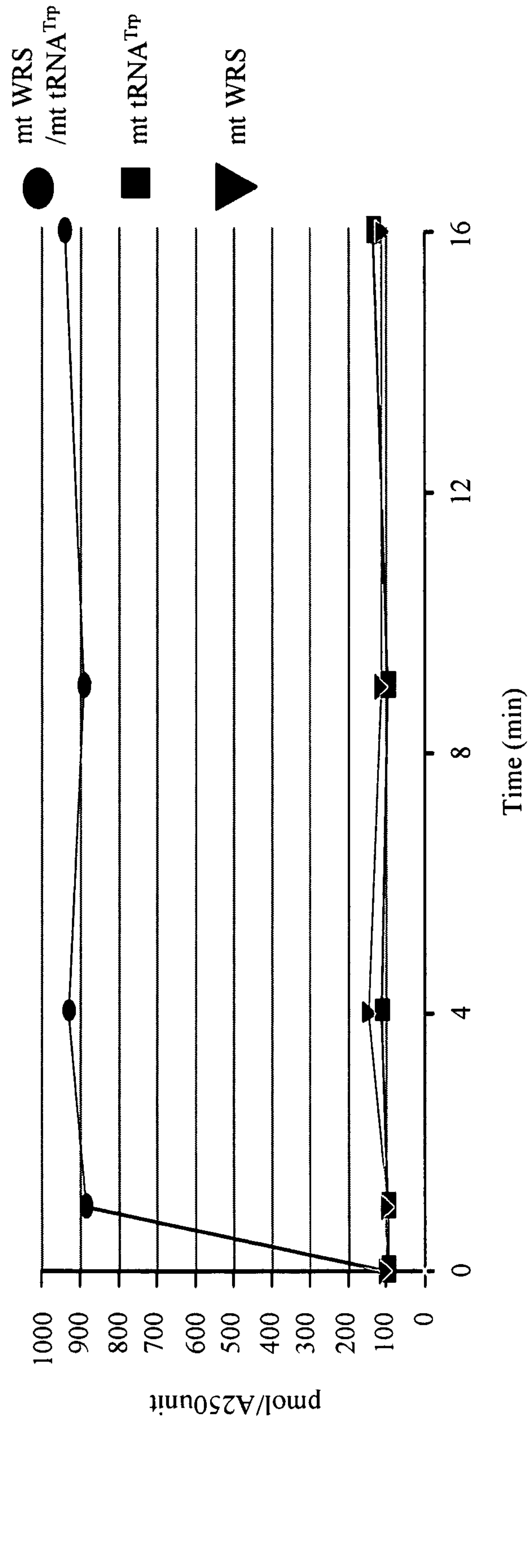

pmol/A250unit
1000
900
800
700
600
500
400
300
200
100
0
0
4
8
12
16
Time (min)
mt WRS /mt tRNA$^{Trp}$
mt tRNA$^{Trp}$
mt WRS

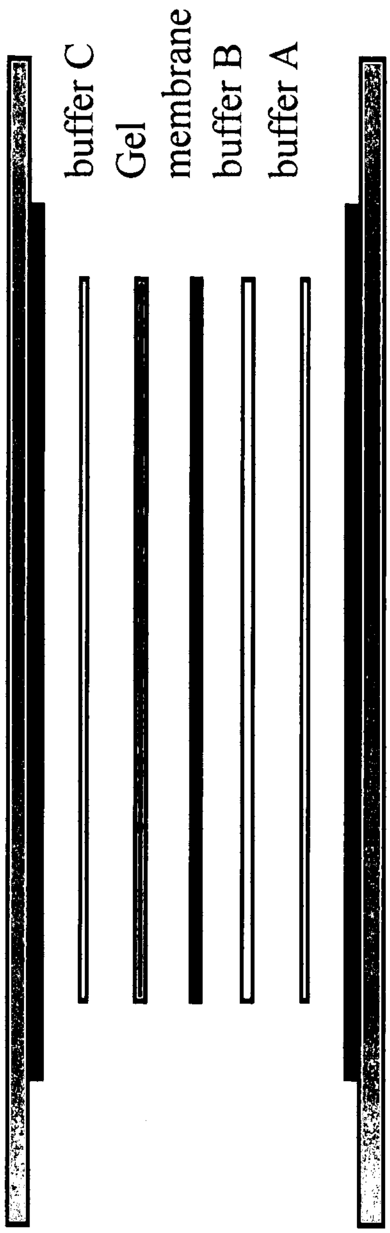


Fig. 12

Fig. 13

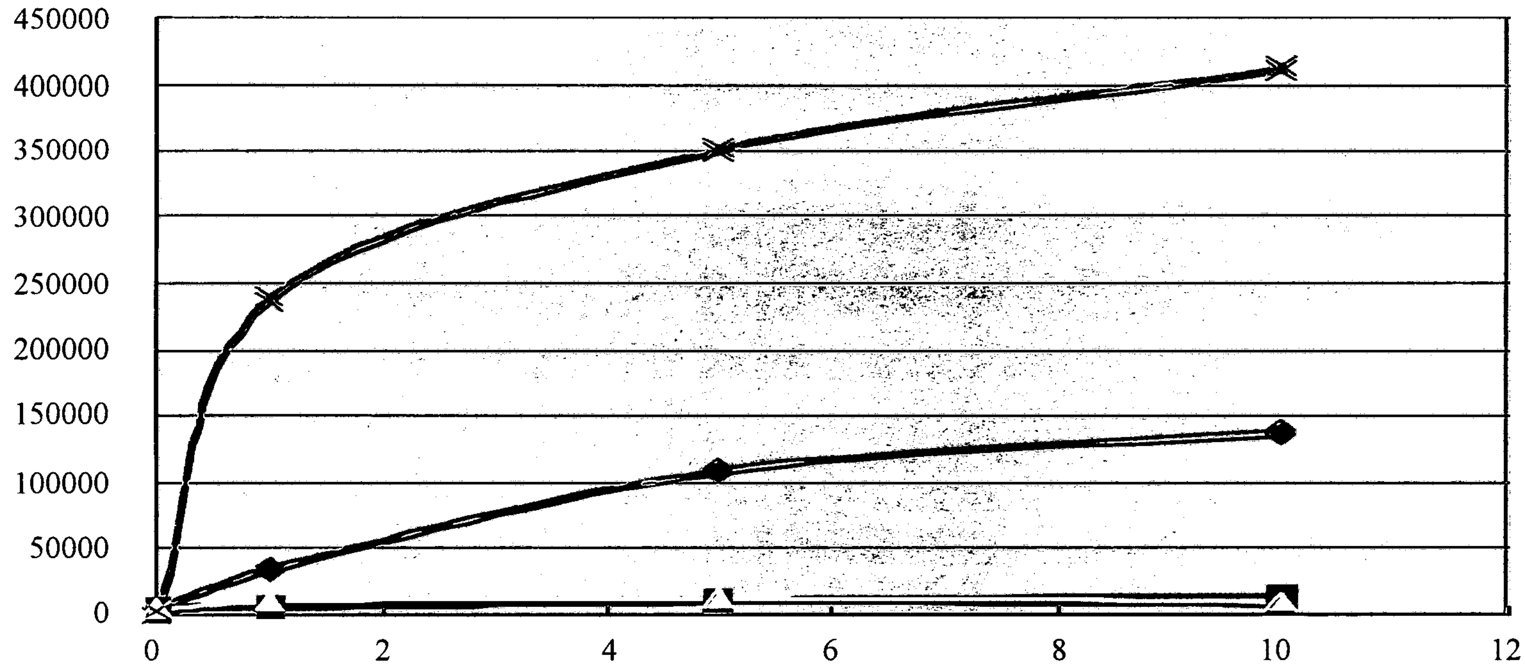


**Confirmation of cross reaction**

Green × : mt WRS and mt $tRNA^{Trp}$

Blue ◆ : wheat germ S100 (without nucleic acid) and wheat tRNAmix

Red ■ : wheat germ S100 (without nucleic acid) and mt $tRNA^{Trp}$

Yellow ▲ : mt WRS and wheat tRNAmix

Fig. 14

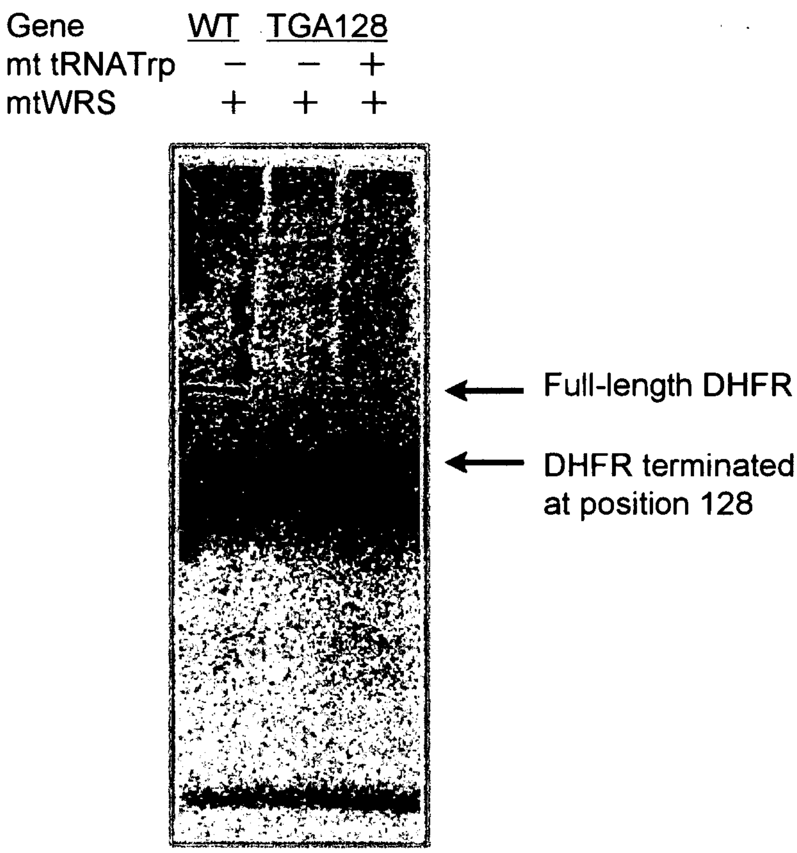
Gene WT TGA128
mt tRNATrp − − +
mtWRS + + +
Full-length DHFR
DHFR terminated at position 128

Fig. 15

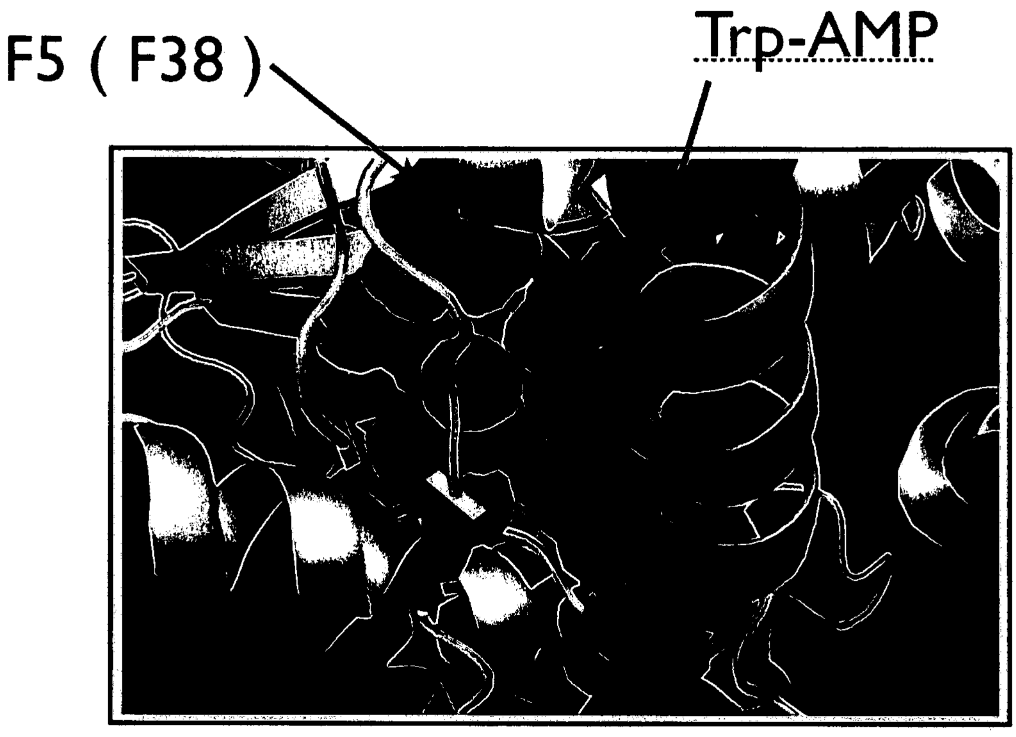

# Fig. 16

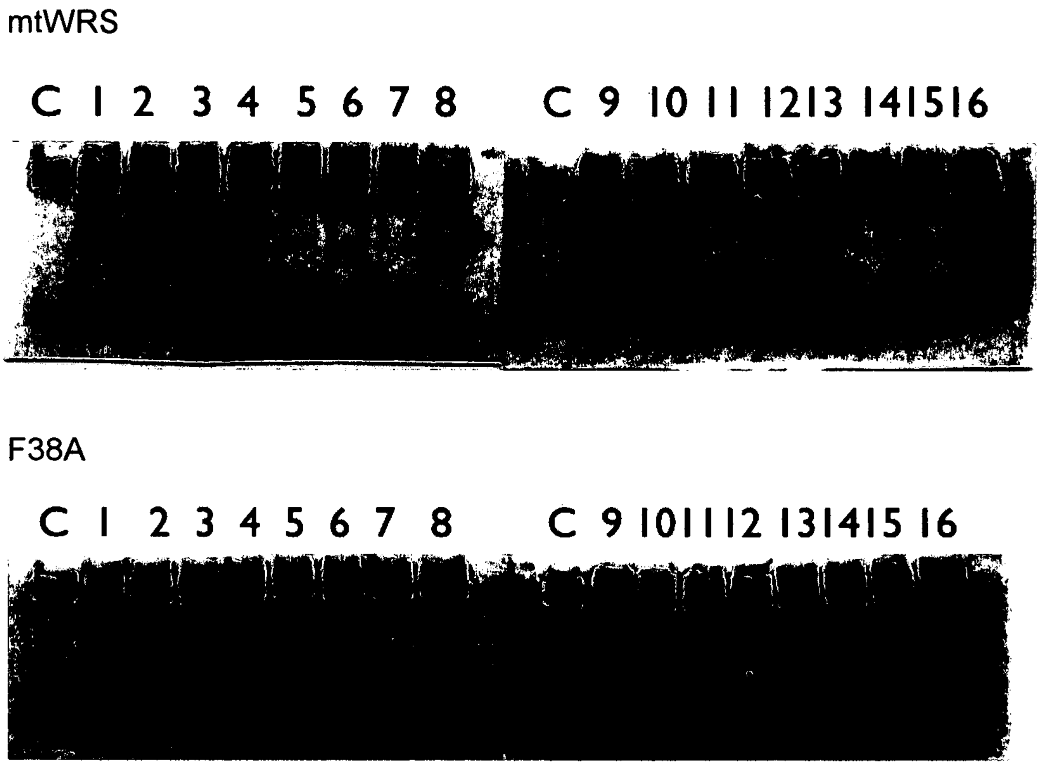


C:EF-Tu,EF-Ts,mt tRNATrp only
1 :5-Me-DL-Trp
2 :5-F-DL-Trp
3 :6-F-DL-Trp
4 :7-Aza-DL-Trp
5 :7-Me-DL-Trp
6 :N α -Me-DL-Trp
7 :3-OH-Kynurenine
8 :5-OH-L-Trp
9 :1-Me-DL-Trp
10:4-F-DL-Trp
11: α -Me-DL-Trp
12:5-MeO-DL-Trp
13:6-Me-DL-Trp
14:Kynurenine
15:3-OH-indole
16:L-Trp

# Fig. 17

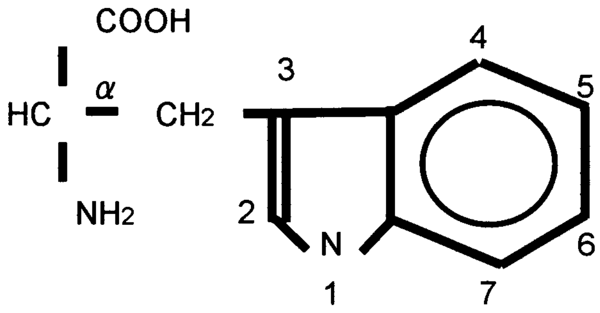

| Position | Functional group | F38A |
|---|---|---|
| α | Me | ○ |
| Nα | Me | — |
| Position 1 | Me | ○ |
| Position 4 | F | △ |
| Position 5 | Me | △ |
| | OH | △ |
| | OMe | — |
| | F | ○ |
| Position 6 | Me | ○ |
| | F | ○ |
| Position 7 | Me | ○ |
| Position 7 | Aza | ○ |

○: acceptance into tRNA
△: some acceptance into tRNA
—: no acceptance into tRNA

**INTERNATIONAL SEARCH REPORT**

International application No.
PCT/JP2005/021938

A. CLASSIFICATION OF SUBJECT MATTER
*C12N15/09*(2006.01), *C07K16/40*(2006.01), *C12N9/00*(2006.01), *C12P19/34*(2006.01), *C12P21/02*(2006.01), *C12Q1/25*(2006.01)

According to International Patent Classification (IPC) or to both national classification and IPC

B. FIELDS SEARCHED

Minimum documentation searched (classification system followed by classification symbols)
*C12N15/09*(2006.01), *C07K16/40*(2006.01), *C12N9/00*(2006.01), *C12P19/34*(2006.01), *C12P21/02*(2006.01), *C12Q1/25*(2006.01)

Documentation searched other than minimum documentation to the extent that such documents are included in the fields searched

Electronic data base consulted during the international search (name of data base and, where practicable, search terms used)
BIOSIS/WPI(DIALOG), GenBank/EMBL/DDBJ/GeneSeq, SwissProt/PIR/Geneseq, JSTPlus(JOIS)

C. DOCUMENTS CONSIDERED TO BE RELEVANT

| Category* | Citation of document, with indication, where appropriate, of the relevant passages | Relevant to claim No. |
|---|---|---|
| X<br>A | JP 2004-261160 A (President of Gifu University), 24 September, 2004 (24.09.04), Full text (Family: none) | 1-5<br>6-16 |
| X<br>A | Myers A.M. et al., MSW, a yeast gene coding for mitochondrial tryptophanyl-tRNA synthetase, J.Biol.Chem., 1985, Vol.260, No.28, pages 15371 to 15377 | 6-10<br>1-5,11-16 |
| X<br>A | Svard S.G. et al., Determinants of Escherichia coli RNase P cleavage site selection: a detailed in vitro and in vivo analysis, Nucleic Acids Res., 1993, Vol.21, No.3, pages 427 to 434 | 11-16<br>1-10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 March, 2006 (13.03.06) | 20 March, 2006 (20.03.06) |
| Name and mailing address of the ISA/<br>Japanese Patent Office | Authorized officer |
| Facsimile No. | Telephone No. |

Form PCT/ISA/210 (second sheet) (April 2005)

INTERNATIONAL SEARCH REPORT

International application No. PCT/JP2005/021938

C (Continuation). DOCUMENTS CONSIDERED TO BE RELEVANT

| Category* | Citation of document, with indication, where appropriate, of the relevant passages | Relevant to claim No. |
|---|---|---|
| X<br>A | Kirsebom L.A., RNase P--a'Scarlet Pimpernel', Mol. Microbiol., 1995, Vol.17, No.3, pages 411 to 420 | 11-16<br>1-10 |
| X<br>A | Guerrier-Takada C. et al., Metal ion requirements and other aspects of the reaction catalyzed by M1 RNA, the RNA subunit of ribonuclease P from Escherichia coli, Biochemistry, 1986, Vol.25, No.7, pages 1509 to 1515 | 11-16<br>1-10 |
| P,X<br>P,A | Katsutoshi FUKUDA et al., "Kobo Mitochondrial Tryptophanyl-tRNA Gosei Koso no Hatsugen to Kino Kaiseki", 27th Annual Meeting of the Molecular Biology Scienty of Japan program Koen Yoshishu, 2004 Dec., Vol.27$^{th}$, page 457, 1PA-244 | 1-10<br>11-16 |

Form PCT/ISA/210 (continuation of second sheet) (April 2005)

**INTERNATIONAL SEARCH REPORT**

International application No.
PCT/JP2005/021938

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
(See extra sheet.)

1. ☒ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet (2)) (April 2005)

**INTERNATIONAL SEARCH REPORT**

International application No.
PCT/JP2005/021938

Continuation of Box No.III of continuation of first sheet(2)

The matter common to claims 1 to 10 resides in "mitochondrial tryptophanyl tRNA synthetase".

As the results of the search, however, it has been clarified that this "mitochondrial tryptophanyl tRNA synthetase" is not novel because of having been reported in documents J.Biol.Chem., 1985, Vol.260, No.28, pages 15371-7 and J.Biol.Chem., 2000, Vol.275, No.22, pages 16820-6.

Consequently, the "mitochondrial tryptophanyl tRNA synthetase" falls within the category of prior art and, therefore, the above common matter cannot be considered as a special technical feature in the meaning within the second sentence of PCT Rule 13.2.

Thus, there is no matter common to all claims.
Since there is no other common matter seemingly being a special technical feature in the meaning within the second sentence of PCT Rule 13.2, no technical relevancy can be found out among these different invention groups in the meaning within PCT Rule 13.

Such being the case, it is obvious that claims 1 to 10 do not comply with the requirement of unity of invention.

The "special technical feature" of claim 1 relates to a method of producing an unnatural amino acid-containing protein wherein an amino acid at a specific site in a protein has been mutated into an unnatural amino acid, while the "special technical feature" of claims 11 to 16 relates to a method of synthesizing tRNA.

Since it does not appear that there is a technical relationship between these groups of inventions involving one or more of the same or corresponding special technical features, these invention groups are not considered as being so linked as to form a single general inventive concept.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 3317983 B
- JP 2004261160 A
- JP 2004338178 A

### Non-patent literature cited in the description


- *JBC,* vol. 257 (11), 6132-6136
- *JBC,* vol. 264, 4304-4311
- *Science,* 1989, vol. 244, 182-188
- *JBC,* vol. 271, 23169
- *Science,* vol. 292, 498-500
- X-ray crystal structural analysis, Expression and preparation of selenomethionine-substituted protein. **TOSHIYUKI SHIMIZU ; KENGO OKADA ; TOSHIO HAKOSHIMA.** KISO SEIKAGAKU JIKKENHO. Tokyo Kagaku Dojin, 15 February 2001, 189-190
- **SAMPSON, J. R. ; UHLENBECK, O. C.** *Proc. Natl. Acad. Sci.,* 1988, vol. 85, 1033-1037
- **KAO, C. ; ZHENG, M. ; RUDISSER, S.** *RNA,* 1999, vol. 5, 1268-1272

SEQUENCE LISTING

<110> Site-directed introduction of unnatural amino acids into proteins by the use of mitochondrial proteins and an effective method for preparation of the tRNAs.

<120> Gifu University

<130> PH-2601-PCT

<140>
<141>

<160> 11

<170> PatentIn Ver. 2.1

<210> 1
<211> 379
<212> PRT
<213> Saccharomyces cerevisiae

<400> 1
Met Ser Asn Lys Gln Ala Val Leu Lys Leu Ile Ser Lys Arg Trp Ile
1 5 10 15

Ser Thr Val Gln Arg Ala Asp Phe Lys Leu Asn Ser Glu Ala Leu His
20 25 30

Ser Asn Ala Thr Val Phe Ser Met Ile Gln Pro Thr Gly Cys Phe His
35 40 45

Leu Gly Asn Tyr Leu Gly Ala Thr Arg Val Trp Thr Asp Leu Cys Glu
50 55 60

Leu Lys Gln Pro Gly Gln Glu Leu Ile Phe Gly Val Ala Asp Leu His
65 70 75 80

Ala Ile Thr Val Pro Lys Pro Asp Gly Glu Met Phe Arg Lys Phe Arg
85 90 95

His Glu Ala Val Ala Ser Ile Leu Ala Val Gly Val Asp Pro Glu Lys
100 105 110

Ala Ser Val Ile Tyr Gln Ser Ala Ile Pro Gln His Ser Glu Leu His
115 120 125

Trp Leu Leu Ser Thr Leu Ala Ser Met Gly Leu Leu Asn Arg Met Thr
130 135 140

Gln Trp Lys Ser Lys Ser Asn Ile Lys Gln Ser Thr Asn Gly Asp Tyr
145 150 155 160

Leu Val Asn Asp Ser Asp Val Gly Lys Val Arg Leu Gly Leu Phe Ser
165 170 175

Tyr Pro Val Leu Gln Ala Ala Asp Ile Leu Leu Tyr Lys Ser Thr His
180 185 190

Val Pro Val Gly Asp Asp Gln Ser Gln His Leu Glu Leu Thr Arg His
195 200 205

Leu Ala Glu Lys Phe Asn Lys Met Tyr Lys Lys Asn Phe Phe Pro Lys
210 215 220

Pro Val Thr Met Leu Ala Gln Thr Lys Lys Val Leu Ser Leu Ser Thr
225 230 235 240

Pro Glu Lys Lys Met Ser Lys Ser Asp Pro Asn His Asp Ser Val Ile
245 250 255

Phe Leu Asn Asp Glu Pro Lys Ala Ile Gln Lys Lys Ile Arg Lys Ala
260 265 270

Leu Thr Asp Ser Ile Ser Asp Arg Phe Tyr Tyr Asp Pro Val Glu Arg
275 280 285

Pro Gly Val Ser Asn Leu Ile Asn Ile Val Ser Gly Ile Gln Arg Lys
290 295 300

Ser Ile Glu Asp Val Val Glu Asp Val Ser Arg Phe Asn Asn Tyr Arg
305 310 315 320

Asp Phe Lys Asp Tyr Val Ser Glu Val Ile Ile Glu Glu Leu Lys Gly
325 330 335

Pro Arg Thr Glu Phe Glu Lys Tyr Ile Asn Glu Pro Thr Tyr Leu His

340 345 350

Ser Val Val Glu Ser Gly Met Arg Lys Ala Arg Glu Lys Ala Ala Lys
355 360 365

Asn Leu Ala Asp Ile His Lys Ile Met Gly Phe
370 375

<210> 2
<211> 364
<212> PRT
<213> Saccharomyces cerevisiae

<400> 2
Ile Ser Thr Val Gln Arg Ala Asp Phe Lys Leu Asn Ser Glu Ala Leu
1 5 10 15

His Ser Asn Ala Thr Val Phe Ser Met Ile Gln Pro Thr Gly Cys Phe
20 25 30

His Leu Gly Asn Tyr Leu Gly Ala Thr Arg Val Trp Thr Asp Leu Cys
35 40 45

Glu Leu Lys Gln Pro Gly Gln Glu Leu Ile Phe Gly Val Ala Asp Leu
50 55 60

His Ala Ile Thr Val Pro Lys Pro Asp Gly Glu Met Phe Arg Lys Phe
65 70 75 80

Arg His Glu Ala Val Ala Ser Ile Leu Ala Val Gly Val Asp Pro Glu
85 90 95

Lys Ala Ser Val Ile Tyr Gln Ser Ala Ile Pro Gln His Ser Glu Leu
100 105 110

His Trp Leu Leu Ser Thr Leu Ala Ser Met Gly Leu Leu Asn Arg Met
115 120 125

Thr Gln Trp Lys Ser Lys Ser Asn Ile Lys Gln Ser Thr Asn Gly Asp
130 135 140

Tyr Leu Val Asn Asp Ser Asp Val Gly Lys Val Arg Leu Gly Leu Phe
145 150 155 160

Ser Tyr Pro Val Leu Gln Ala Ala Asp Ile Leu Leu Tyr Lys Ser Thr
165 170 175

His Val Pro Val Gly Asp Asp Gln Ser Gln His Leu Glu Leu Thr Arg
180 185 190

His Leu Ala Glu Lys Phe Asn Lys Met Tyr Lys Lys Asn Phe Phe Pro
195 200 205

Lys Pro Val Thr Met Leu Ala Gln Thr Lys Lys Val Leu Ser Leu Ser
210 215 220

Thr Pro Glu Lys Lys Met Ser Lys Ser Asp Pro Asn His Asp Ser Val
225 230 235 240

Ile Phe Leu Asn Asp Glu Pro Lys Ala Ile Gln Lys Lys Ile Arg Lys
245 250 255

Ala Leu Thr Asp Ser Ile Ser Asp Arg Phe Tyr Tyr Asp Pro Val Glu
260 265 270

Arg Pro Gly Val Ser Asn Leu Ile Asn Ile Val Ser Gly Ile Gln Arg
275 280 285

Lys Ser Ile Glu Asp Val Val Glu Asp Val Ser Arg Phe Asn Asn Tyr
290 295 300

Arg Asp Phe Lys Asp Tyr Val Ser Glu Val Ile Ile Glu Glu Leu Lys
305 310 315 320

Gly Pro Arg Thr Glu Phe Glu Lys Tyr Ile Asn Glu Pro Thr Tyr Leu
325 330 335

His Ser Val Val Glu Ser Gly Met Arg Lys Ala Arg Glu Lys Ala Ala
340 345 350

Lys Asn Leu Ala Asp Ile His Lys Ile Met Gly Phe
355 360

<210> 3
<211> 1140
<212> DNA

<213> Saccharomyces cerevisiae

<400> 3
atgtcgaata agcaggcggt tctgaagtta atcagtaaaa ggtggataag cacagtgcaa 60
cgtgccgatt ttaagctgaa ttccgaagcg cttcatagta atgctacggt atttagtatg 120
attcagccaa ctgggtgttt ccaccttggt aattatctag gtgctacacg tgtttggaca 180
gacttatgtg aattaaaaca acctggccag gaattgatat ttggagttgc tgatttacac 240
gctatcactg ttccaaagcc agatggagaa atgtttagaa aatttcgcca tgaggcagta 300
gcaagtatac tggctgtcgg tgtagatcca gaaaaggctt cggtgatcta ccaatccgcc 360
atccctcaac acagtgaatt acactggttg ctttctactc ttgcctcgat gggattactt 420
aaccgcatga cgcaatggaa atcaaaatcg aatatcaaac aatcaactaa tggggattat 480
ctggttaacg attctgacgt gggaaaagtt agactgggct tattttcgta tcctgtttta 540
caggcagcgg atattctact ttataaatct acacacgttc ctgttggcga tgatcaatcg 600
cagcatttgg aactaacaag acaccttgct gaaaagttta acaaaatgta caagaaaaat 660
tttttcccta aacctgtaac tatgttggca caaacgaaaa aagttttgag cttaagcacg 720
cctgaaaaga aaatgtccaa gagtgatccg aatcacgact ctgtaatttt tttgaatgat 780
gagcctaagg caattcagaa aaagattaga aaagctttaa cagattccat ttctgatagg 840
ttctactacg accctgtaga aagaccaggc gtatcgaatt taattaacat tgtcagtggc 900
attcaaagaa aatcgattga agatgtcgta gaagatgtat ctcgtttcaa taactatagg 960
gatttcaaag attatgtttc agaagtgata attgaggaat tgaaaggccc aagaacagaa 1020
tttgagaaat atatcaacga accgacctat ttgcatagtg tcgttgaatc tggcatgcgc 1080
aaagcgagag aaaaagcagc aaaaaacctg gccgacattc ataaaataat gggcttctga 1140

<210> 4
<211> 1095
<212> DNA
<213> Saccharomyces cerevisiae

<400> 4
ataagcacag tgcaacgtgc cgattttaag ctgaattccg aagcgcttca tagtaatgct 60
acggtattta gtatgattca gccaactggg tgtttccacc ttggtaatta tctaggtgct 120
acacgtgttt ggacagactt atgtgaatta aaacaacctg gccaggaatt gatatttgga 180
gttgctgatt tacacgctat cactgttcca aagccagatg gagaaatgtt tagaaaattt 240
cgccatgagg cagtagcaag tatactggct gtcggtgtag atccagaaaa ggcttcggtg 300
atctaccaat ccgccatccc tcaacacagt gaattacact ggttgctttc tactcttgcc 360
tcgatgggat tacttaaccg catgacgcaa tggaaatcaa aatcgaatat caaacaatca 420
actaatgggg attatctggt taacgattct gacgtgggaa aagttagact gggcttattt 480
tcgtatcctg ttttacaggc agcggatatt ctactttata aatctacaca cgttcctgtt 540
ggcgatgatc aatcgcagca tttggaacta acaagacacc ttgctgaaaa gtttaacaaa 600
atgtacaaga aaattttttt tcctaaacct gtaactatgt tggcacaaac gaaaaaagtt 660
ttgagcttaa gcacgcctga aaagaaaatg tccaagagtg atccgaatca cgactctgta 720
atttttttga atgatgagcc taaggcaatt cagaaaaaga ttagaaaagc tttaacagat 780
tccatttctg ataggttcta ctacgaccct gtagaaagac caggcgtatc gaatttaatt 840

aacattgtca gtggcattca aagaaaatcg attgaagatg tcgtagaaga tgtatctcgt 900
ttcaataact atagggattt caaagattat gtttcagaag tgataattga ggaattgaaa 960
ggcccaagaa cagaatttga gaaatatatc aacgaaccga cctatttgca tagtgtcgtt 1020

gaatctggca tgcgcaaagc gagagaaaaa gcagcaaaaa acctggccga cattcataaa 1080
ataatgggct tctga 1095

<210> 5
<211> 48
<212> DNA
<213> Artificial Sequence

<220>
<223> Description of Artificial Sequence:primer

<400> 5
gggggatcc aaaaaaatgt ctaataagca ggcggttctg aagttaat 48

<210> 6
<211> 42
<212> DNA
<213> Artificial Sequence

<220>
<223> Description of Artificial Sequence:primer

<400> 6
ggggaccggt ctcgaggaag cccattattt tatgaatgtc gg 42

<210> 7
<211> 23
<212> DNA
<213> Artificial Sequence

<220>
<223> Description of Artificial Sequence:primer

<400> 7
gggtctagaa aggatatagt tta 23

<210> 8
<211> 26
<212> DNA
<213> Artificial Sequence

<220>
<223> Description of Artificial Sequence:primer

<400> 8
gggaagcttc ctggcaagga taaaga 26

<210> 9
<211> 19
<212> DNA
<213> Artificial Sequence

<220>
<223> Description of Artificial Sequence:primer

<400> 9
tngcaaggat aaagagatt 19

<210> 10
<211> 27
<212> DNA
<213> Artificial Sequence

<220>
<223> Description of Artificial Sequence:primer

<400> 10
ggggctgcag taatacgact cactata 27

<210> 11
<211> 74
<212> DNA
<213> Saccharomyces cerevisiae

<400> 11
aaggatatag tttaatggta aaacagttga tttcaaatca atcattagga gttcgaatct 60
ctttatcctt gcca 74